# EUROPEAN PATENT SPECIFICATION

(11) **EP 2 679 226 B1**
(45) Date of publication and mention of the grant of the patent: **06.04.2016**
(21) Application number: 12749626.3
(22) Date of filing: 23.02.2012
(51) Int. Cl.: A61K 31/352, A61P 27/02, A61P 27/04, A61P 43/00, C07D 311/86

(54) **THERAPEUTIC AGENT FOR CORNEAL SENSORY NERVE DAMAGE CONTAINING SEMAPHORIN INHIBITOR AS ACTIVE INGREDIENT**
THERAPEUTISCHES MITTEL GEGEN SENSORISCHE HORNHAUTNERVENSCHÄDEN MIT EINEM SEMAPHORINHEMMER ALS WIRKSTOFF
AGENT THÉRAPEUTIQUE POUR UNE LÉSION DE NERF SENSORIEL CORNÉEN CONTENANT UN INHIBITEUR DE LA SÉMAPHORINE EN TANT QUE PRINCIPE ACTIF

(30) Priority: 25.02.2011 JP 2011040128
(43) Date of publication of application: 01.01.2014
(73) Proprietor: Keio University, Tokyo 108-8345 (JP); Sumitomo Dainippon Pharma Co., Ltd., Osaka-shi Osaka 541-0045 (JP)
(72) Inventor: OKANO, Hideyuki, Tokyo 160-8582 (JP); TSUBOTA, Kazuo, Tokyo 160-8582 (JP); SHIMMURA, Shigeto, Tokyo 160-8582 (JP); OMOTO, Masahiro, Tokyo 160-8582 (JP); KISHINO, Akiyoshi, Osaka-shi Osaka 554-0022 (JP); MAEDA, Miho, Suita-shi Osaka 564-0053 (JP)
(74) Representative: Duckworth, Timothy John
(86) International application number: PCT/JP2012/054394
(87) International publication number: WO 2012/115182

(56) References cited:
- EP-A1- 1 306 093
- JP-A- 2006 335 683
- JP-A- 2008 013 530
- MORISHIGE,N. ET AL.: 'Expression of semaphorin 3A in the rat corneal epithelium during wound healing' BIOCHEM BIOPHYS RES COMMUN vol. 395, no. 4, 14 May 2010, pages 451 - 457, XP027048299
- TANELIAN,D.L. ET AL.: 'Semaphorin III can repulse and inhibit adult sensory afferents in vivo' NAT MED vol. 3, no. 12, 03 December 1997, pages 1398 - 1401, XP008170312

## Description

### Technical Field

The present invention relates to a therapeutic agent for sensory neuropathy caused by corneal disease or corneal surgery, or dry eyes associated therewith, wherein the therapeutic agent comprises, as an active agent, a xanthone compound having a semaphorin inhibitory activity.

### Background Art

The cornea is a transparent membrane that covers the front of the eye, and it has a function to introduce light into the eye and then to refract the light so as to focus the eyes together with the lens. Moreover, since the surface of the cornea is always covered with tears, the cornea also has a function to prevent dry eyes or bacterial infections in the eyes. This cornea may be weakened or degenerated due to injury or disease, and as a result, the above-mentioned functions may be lost. In order to treat such symptoms, a drug is first used. In addition, in a case in which such a drug does not bring on sufficient therapeutic effects, transplantation of a normal cornea into the weakened or degenerated cornea is performed.

The corneal sensory nerve, which is a sensory nerve derived from the trigeminal nerve, is distributed on the surface layer of the cornea. The trigeminal nerve is one of the cranial nerves, and is also referred to as the fifth cranial nerve. The trigeminal nerve branches into three nerves, namely, ophthalmic nerve, maxillary nerve and mandibular nerve. Among the ophthalmic nerve, the nerve traveling in the cornea is the corneal sensory nerve. The corneal sensory nerve plays not only a role for causing corneal reflection (blink reflection) by corneal sensitivity to protect the cornea, but also a role for producing tears and promoting the secretion of neurotrophic factors to maintain corneal homeostasis.

In corneal disease, not only degeneration of the cornea itself, but also corneal sensory nerve damage also occurs, and thus, a normal nerve function is impaired and sensory disturbance takes place. Moreover, in corneal surgery including, as typical examples, keratoplasty for the treatment of diseases or myopia correction surgery such as LASIK, since corneal sensory nerve is disconnected, sensory disturbance must occur for a long period of time after completion of the surgery. Such sensory disturbance, namely, corneal sensory neuropathy specifically means reduction in sensory perception such as tactile perception or pain perception on the corneal surface, and it causes the lack of corneal reflection, dry eyes due to abnormality in tears (corneal xerosis), damage on eyeballs, etc.

Examples of the corneal disease known as a cause of corneal sensory neuropathy or dry eyes include keratitis, leukoma (which is caused by corneal herpes, measles, syphilis, or injury), corneal infection, corneal degeneration, corneal dystrophy, corneal stromal dystrophy, bullous keratopathy, keratoconus, corneal decompensation, corneal ulcer, neuroparalytic keratopathy, diabetic keratopathy, chemical burns of the cornea (which is caused by a chemical or the like that has entered into the eyes), and thermal burns of the cornea. When the disease is severe, keratoplasty may be applied to treat it.

Examples of the corneal surgery known as a cause of corneal sensory neuropathy or dry eyes include keratoplasty, myopia correction surgery, and an interposition operation to insert artificial lens (artificial crystalline lens) for the treatment of cataract and the like.

Keratoplasty is also referred to as corneal transplantation or corneal plasty. The practical application of keratoplasty is not limited to the aforementioned cases. The purposes of application of keratoplasty include 1) an optical purpose, 2) a therapeutic purpose, 3) a surgical purpose, and 4) a cosmetic purpose.

The optical purpose in 1) above means restoration of transparency of the cloudy cornea and recovery of vision. Examples of a causative disease for these symptoms include keratitis, leukoma (which is caused by corneal herpes, measles, syphilis, or injury), corneal infection, corneal degeneration, corneal dystrophy, corneal stromal dystrophy, bullous keratopathy, keratoconus, and corneal decompensation. The therapeutic purpose in 2) above means alleviation of infection by ablation of the cornea as a focus of infection. An example of a causative disease for this symptom is corneal ulcer (mainly, the infection in an active stage). The surgical purpose in 3) above means retention of the shape of an eyeball in the case of corneal perforation. Examples of a causative disease for these symptoms include corneal ulcer (bacterial, fungal, viral or sterile ulcer) and injury. The cosmetic purpose in 4) above means the cosmetic improvement of leukoma associated with the cloudiness of the cornea.

Specific examples of keratoplasty include 1) penetrating keratoplasty, 2) lamellar keratoplasty, 3) deep lamellar keratoplasty, 4) sclerokeratoplasty, 5) scleral graft transplantation, 6) limbus corneae transplantation, and 7) amnion transplantation.

The penetrating keratoplasty in 1) above is an operation for exchanging the full layer ranging from the ectocornea to the endothelium corneae. This transplantation is used for the treatment of a disease involving cloudiness that reaches the deep layer of the parenchyma of cornea, such as a bullous corneal disease that needs endothelial transplantation or ulcer perforation. The lamellar keratoplasty in 2) above is an operation for excising only the ectocornea and a lesion in the parenchyma of cornea and then transplanting therein a corneal section of the same size as the excised portion. This transplantation is used in a case in which only the surface layer of the parenchyma of cornea becomes clouded, in a case in which the peripheral portion of the cornea becomes thin, and in a case in which the cornea locally becomes thin. The deep lamellar keratoplasty in 3) above is an operation for excising the ectocornea and the parenchyma of cornea as a whole, while only the Demes membrane and the endothelium corneae are left, and then transplanting only the ectocornea and the parenchyma of cornea adjusted to have the same size as the excised portion. This transplantation is used in a case in which endothelial cells are healthy. The sclerokeratoplasty in 4) above is an operation for excising the cornea as well as the sclera and then transplanting a sclerocorneal section into the excised portion. This transplantation is used, for example, in a case in which corneal ulcer is spread over a wide range. The scleral graft transplantation in 5) above is an operation for correcting the thinned sclera. This transplantation is used in a case in which the cornea is healthy and the sclera is reinforced. The limbus corneae transplantation in 6) above is an operation for transplanting the stem cells of the limbus corneae in order to supply a normal ectocornea. The amnion transplantation in 7) above is an operation for excising an abnormal conjunctiva and then transplanting an amnion into the excised portion. As a result of this operation, the environment is adjusted, so that an eyeball can be covered again with a normal conjunctiva.

Examples of causative diseases that require keratoplasty at a high frequency in Japan include: firstly, keratoconus; secondly, leukoma; thirdly, bullous keratopathy; fourthly, corneal degeneration; and fifthly, chemical burns of the cornea or thermal burns of the cornea. Keratoconus is a corneal dystrophy which develops at the time of puberty. In this disease, the central portion of the cornea is gradually thinned and is projected forwards. Since the shape of the cornea is deformed, the function of the cornea as lens is impaired. In a case in which keratoconus is severe, and thus, vision cannot be sufficiently corrected with contact lens or it is difficult to use contact lens for a long period of time, keratoplasty may be carried out. Leukoma is frequently developed in aged people, and it indicates cicatrix remaining on the cornea as a result of being affected with keratitis in early life. Cloudiness occurring after herpetic keratitis is also included in leukoma. Bullous keratopathy indicates a condition in which endothelial cells for regulating water content in the cornea in the back side thereof are decreased and as a result, water is accumulated and the cornea is swollen. A condition in which endothelial cells are decreased in a patient who has previously undergone keratoplasty, and in which the patient needs keratoplasty again, which is referred to as replantation, is also included in bullous keratopathy. Corneal degeneration indicates a condition in which abnormal substances are sedimented in the cornea and become clouded. Chemical burns of the cornea and thermal burns of the cornea indicate a condition in which a chemical or cement entered in eyes and as a result, a strong cicatrix is generated.

It is said that, for example, 1,000 to 2,000 cases of such keratoplasty are carried out on a year basis in Japan. It is also said that the number of patients who require keratoplasty are about 20,000 for a year basis.

Other than keratoplasty, myopia correction surgery is an operation in which corneal sensory neuropathy may occur as a result of the disconnection of the corneal sensory nerve. The term "myopia correction surgery" is mainly used to mean an operation for recovering vision decreased due to myopia.

Specific examples of the operation that is collectively referred to as myopia correction surgery at current include radial keratotomy (RK), photorefractive keratectomy (PRK), and laser in situ keratomileusis (LASIK, keratorefractive surgery). Among these operations, excimer laser is used in PRK and LASIK. Ten years ago, the main myopia correction surgery was radial keratotomy. However, recently, the most common myopia correction surgery is LASIK. Hence, the trend of myopia correction surgery has been recently changed in a short time, and thus it can be said that the most-advanced operation method will be replaced with a new operation method. In the case of LASIK for example, 50,000 to 60,000 cases of LASIK operations are carried out for a year basis in Japan. The number of cases of requiring LASIK operations tends to be increased. With such an increase in the number of LASIK operations, dry eyes and the like have been reported as aftereffects of LASIK.

Moreover, myopia correction surgery also includes myopia correction methods other than the surgery, such as LASEK, Intrastromal Corneal Ring Segments (ICRS), intracorneal lens, phakic intraocular lens, and orthokeratology. The same corneal sensory neuropathy as described above has been developed also from these myopia correction methods.

Furthermore, not only by keratoplasty and myopia correction surgery, but the corneal sensory nerve is also disconnected by surgical operations that target the cornea required for the treatment of eye disease or corneal injury, such as keratotomy, keratectomy, keratorefractive surgery, orthokeratologic procedure or keratomilusis. As a result, the same sensory neuropathy as described above is developed by these surgical operations. Further, in the treatment of cataract and the like, an operation may be carried out to insert artificial lens into the eyes. Even in such an operation, since the cornea is slightly incised, it is likely that the corneal sensory nerve may be damaged.

Semaphorin is an endogenous protein that was identified as a factor for causing the nerve growth cone to degenerate and suppressing the elongation of axon. To date, approximately 20 types of molecular species have been known. Among these species, a group of genes, which is referred to as the class 3 subfamily, has been well studied, and it has been known that proteins encoded by these genes have a strong in vitro neurite elongation-suppressing activity or an activity of causing the growth cone to degenerate. The most studied factor is semaphorin 3A(Sema3A, collapsin-1) (Non Patent Literatures 1 and 2), and this protein induces the growth cones of cultured nerve cells to degenerate at a low concentration in a short time.

As substances having a semaphorin inhibitory activity against semaphorin, there have been known: a series of xanthone compounds (Patent Literatures 1 and 2) obtained from the cultures of Penicillium sp. SPF-3059 strain (Accession No. FERM BP-7663; NITE Patent Microorganisms Depositary (NPMD)); and derivatives formed by chemically modifying such xanthone compounds (Patent Literature 3). The xanthone compound has an action to promote neurogeneration in vivo. Moreover, it has also been reported that such a xanthone compound suppresses nerve cell death associated with ischemic disorder and is effective for the treatment or prevention of ischemic neuropathy (Patent Literature 4).

The action of semaphorin on the sensory nerve of an adult animal in vivo has been reported (Non Patent Literature 3). Specifically, it has been reported that, when a semaphorin 3 gene is introduced into rabbit ectocorneal cells using a gene gun, the trigeminal nerve is degenerated, and that the re-elongation of the trigeminal nerve is suppressed by administration of a Sema3 gene to an adult rabbit corneal wound model in which the ectocornea has been exfoliated and removed. This report suggests that the sensory nerve of an adult be agonistically adjusted by a Sema3 gene and that chronic pain be treated by administration of such a Sema3 gene. However, this report neither discloses nor suggests a method for promoting the regeneration of the corneal sensory nerve that has been disconnected due to corneal damage. This is because the report does not suggest that the corneal sensory nerve cannot be sufficiently regenerated if no treatments are performed after corneal damage. In addition, it has not become clear that semaphorin that is expressed in the cornea causes insufficient regeneration of the corneal sensory nerve.

It has been reported that semaphorin 3A derived from lens repulsively controls the formation of the corneal sensory nerve during the developmental period (fetal life) (Non Patent Literature 4). In this publication, it is considered that the sensory nerve of an eyeball is generally formed during a fetal life such that it avoids the cornea, thereby forming a circular nerve ring, but that such a circular nerve ring cannot be normally formed when crystalline lens are eliminated from a chicken embryo, and thus that a factor that repulsively acts on nerve elongation is secreted from the crystalline lens. According to this publication, when the crystalline lens was co-cultured with the sensory nerve, the sensory nerve was not elongated in a direction in which the crystalline lens was present. However, the sensory nerve was elongated in the aforementioned direction by addition of a Sema3A-blocking peptide. Accordingly, it has been considered in the aforementioned publication that the factor that repulsively acts on the elongation of the sensory nerve is semaphorin 3A derived from the crystalline lens. Moreover, when a Sema3A-blocking peptide was added into or around the crystalline lens of a normal embryo, the elongation of the corneal sensory nerve was confirmed. Based on these results, this publication has reported that crystalline lens-derived semaphorin 3A repulsively controls the formation of the corneal sensory nerve during the developmental period (fetal life). However, this publication neither discloses nor suggests a method for promoting the regeneration of the corneal sensory nerve that has been disconnected due to corneal damage. The gist of this publication is to elucidate the mechanism of the characteristic formation of the corneal sensory nerve during the developmental period. Therefore, this publication neither describes the association of semaphorin with disease, nor studies semaphorin using pathological condition models. This publication has clarified using a Sema3A-blocking peptide that the sensory nerve does not enter into the cornea because semaphorin 3A repulsively acts thereon. However, this is the finding obtained in the case of the formative period of the normal nerve in an embryo in the developmental period (fetal life). The circumstances are radically different from the case of inhibiting the elongation of the corneal sensory nerve in the damaged cornea. Moreover, in this publication, an inhibitory experiment has not been carried out using corneal damage models. Thus, the elongation of the corneal sensory nerve in the damaged cornea cannot be analyzed. Furthermore, only a chicken embryo has been used as an in vivo model, and rodents have not been used in the studies. Accordingly, this report neither discloses nor suggests a method for promoting the regeneration of the corneal sensory nerve that has been disconnected due to corneal damage.

It has been reported that the fact that semaphorin 3 A is expressed and present in the cornea of rats had been demonstrated by an immunostaining method or a real-time PCR method (Non Patent Literature 5). However, this report neither discloses nor suggests a method for promoting the regeneration of the corneal sensory nerve that has been disconnected due to corneal damage. In this publication, no inhibitory experiments have been carried out, no experiments have been carried out using damage models or pathological condition models, and there are no descriptions regarding corneal damage.

It has been reported that the fact that, in the case of mice, semaphorin 3 A and the receptors thereof, namely, neuropilin-1 and plexinA, are expressed and present in the cornea in a period from the fetal life to at least two weeks after the birth, had been demonstrated by an immunostaining method or a real-time PCR method (Non Patent Literature 6). However, the gist of this report is the association of semaphorin 3A with the formation of the cornea in a period from the fetal life to the time immediately after the birth. This publication neither discloses nor suggests a method for promoting the regeneration of the corneal sensory nerve that has been disconnected due to corneal damage. In this publication, no inhibitory experiments have been carried out, no experiments have been carried out using damage models or pathological condition models, and there are no descriptions regarding corneal damage.

It has been reported that the fact that a fluctuation in the expression of semaphorin 3A is associated with the formation of the corneal nerve in the fetal life of a chicken had been demonstrated by an in situ hybridization method or a real-time PCR method (Non Patent Literature 7). However, this report includes not a report about mammals, but a report about birds. In addition, the gist of this publication is to elucidate the mechanism of formation of the corneal nerve circuit in the fetal life. This publication neither discloses nor suggests a method for promoting the regeneration of the corneal sensory nerve that has been disconnected due to corneal damage. Moreover, in this publication, no inhibitory experiments have been carried out, no experiments have been carried out using damage models or pathological condition models, and there are no descriptions regarding corneal damage.

/ A fluctuation in the expression of semaphorin 3A and its receptor neuropilin-1 has been examined using adult rat corneal wound models. As a result, it was found that the expression levels of semaphorin 3A and neuropilin-1 were increased over the entire layer of the edge portion of a wound area due to damage, but that the expression levels returned to normal levels as a result of healing. Thus, it is suggested that semaphorin 3A be deeply associated with the healing of the wound in the ectocornea (Non Patent Literature 8). However, this publication is relevant to the regeneration of ectocorneal cells, and it neither discloses nor suggests a method for promoting the regeneration of the corneal sensory nerve that has been disconnected due to corneal damage. These damage models are different from damage models due to keratoplasty or sensory nerve damage. In this publication, no inhibitory experiments have been carried out, and there are no descriptions regarding sensory nerve function.
JP-A-2006/335683 discloses xanthone compounds having semaphorin-inhibiting activities useful as nerve regeneration promoters in the centre and periphery and as a prophylactic/therapeutic agent for neuropathic disease, neurodegeneration disease and neurological disease accompanying ischemic injury such as glaucoma.
JP-A-2008/013530 discloses a method for producing xanthone compounds having semaphorin-inhibiting activity.

### Citation List

### Patent Literature

Patent Literature 1: International Publication WO02/09756
Patent Literature 2: International Publication WO03/062243
Patent Literature 3: International Publication WO03/062440
Patent Literature 4: International Publication WO 2005/053678

### Non Patent Literature

Non Patent Literature 1: Cell, Volume 75, p. 217, 1993
Non Patent Literature 2: Cell, Volume 75, p. 1389, 1993
Non Patent Literature 3: Nature Medicine, Volume 3, Number 12, p. 1398, 1997
Non Patent Literature 4: Development Biology, Volume 306, Number 2, p. 750, 2007
Non Patent Literature 5: Experimental Eye Research, Volume 86, Number 4, p. 669, 2008
Non Patent Literature 6: Biochemical and Biophysical Research Communications, Volume 403, Number 3-4, p. 305, 2010
Non Patent Literature 7: Developmental Biology, Volume 344, Number 1, p. 172, 2010
Non Patent Literature 8: Biochemical and Biophysical Research Commmunications, Volume 395, Number 4, p. 451, 2010

### Summary of Invention

### Technical Problem

As described above, there have been many reports about studies regarding cornea and semaphorin. However, no studies have been conducted regarding the fact that a xanthone compound having a semaphorin 3A inhibitory activity promotes the re-elongation of the corneal sensory nerve damaged or disconnected due to corneal disease or corneal surgery such as keratoplasty and has the effect of a therapeutic or preventive agent effective for sensory neuropathy caused by corneal disease or corneal surgery. This fact has not been known up to now.

Accordingly, it is an object of the present invention to provide a therapeutic or preventive agent for sensory neuropathy caused by corneal disease or corneal surgery, comprising, as an active ingredient, a xanthone compound having a semaphorin 3A inhibitory activity. It is another object of the present invention to provide a promoter for the regeneration of the corneal sensory nerve, comprising, as an active ingredient, a xanthone compound having a semaphorin 3 A inhibitory activity.

### [Solution to Problem].

The corneal sensory nerve is damaged by corneal disease or keratoplasty and thereby causes sensory neuropathy. Since the corneal sensory nerve is necessarily disconnected by keratoplasty, it is considered to be the most severe model of the corneal sensory nerve damage. The corneal sensory nerve cannot be sufficiently re-elongated in the transplanted cornea, and as a result, sensory disturbance occurs. The present inventors have thought that the action of semaphorin 3A expressing on the lens or ectocornea would cause such insufficient re-elongation of the corneal sensory nerve. The inventors have conducted intensive studies regarding whether or not the use of a xanthone compound having a semaphorin 3A inhibitory activity enables the re-elongation of the corneal sensory nerve and the improvement of sensory disturbance. As a result, the present inventors have elucidated for the first time that such a xanthone compound having a semaphorin 3A inhibitory activity promotes regeneration of the corneal sensory nerve in a mouse keratoplasty model. Thus, the inventors have found that such a xanthone compound having a semaphorin 3A inhibitory activity is effective as a therapeutic agent or a preventive agent for corneal sensory neuropathy caused by the corneal sensory nerve damaged due to corneal disease or corneal surgery. The present invention has been completed as a result of intensive studies further conducted based on the above-mentioned findings.

Specifically, the present invention relates to the following [1] to [20]:
[1] An agent comprising, as an active ingredient, a compound represented by the following formula (1) or a pharmaceutically acceptable salt thereof: 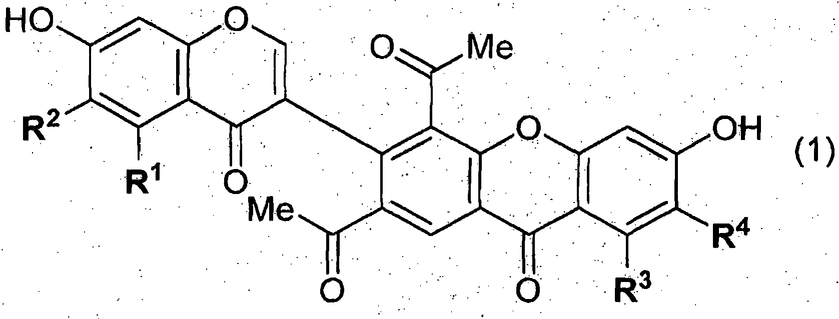 wherein R¹ represents a hydrogen atom or a carboxyl group; R² represents a hydrogen atom or a hydroxyl group; R³ represents a hydrogen atom or a carboxyl group; and R⁴ represents a hydrogen atom or a hydroxyl group;
   for use in a method that is:
   (i) a method for treatment or prevention of sensory neuropathy caused by corneal disease or corneal surgery; or
   (ii) a method for promoting regeneration of corneal sensory nerve;
   wherein the compound represented by the formula (1) or a pharmaceutically acceptable salt thereof is parenterally administered topically into eyes;
[2] The agent for use according to [1], wherein the corneal sensory nerve has been disconnected or damaged in the sensory neuropathy;
[3] The agent for use according to [1] or [2], wherein the method is a method for promoting regeneration of corneal sensory nerve and a method for treatment or prevention of sensory neuropathy caused by corneal disease or corneal surgery;
[4] The agent for use according to [1], [2] or [3] above, wherein, in the formula (1), R¹ represents a hydrogen atom or a carboxyl group, R² represents a hydrogen atom or a hydroxyl group, R³ represents a hydrogen atom or a carboxyl group, and R⁴ represents a hydrogen atom or a hydroxyl group;
[5] The agent for use according to any one of [1] or [4] above, wherein, in the formula (1), at least one of R² and R⁴ represents a hydroxyl group;
[6] The agent for use according to [5] above, wherein, in the formula (1), R² represents a hydroxyl group;
[7] The agent for use according to [5] above, wherein, in the formula (1), R² and R⁴ each represent a hydroxyl group;
[8] The agent for use according to any one of [1] to [7] above, wherein, in the formula (1), at least one of R¹ and R³ represents a carboxyl group;
[9] The agent for use according to [8] above, wherein, in the formula (1), R³ represents a carboxyl group;
[10] The agent according to any one of [1] to [3] above, wherein, in the formula (1), R¹ and R³ each represent a carboxyl group, and R² and R⁴ each represent a hydroxyl group;
[11] The agent for use according to any one of [1] to [10] above, wherein the sensory neuropathy is caused by corneal disease and the corneal disease is keratitis, leukoma, corneal infection, corneal degeneration, corneal dystrophy, corneal stromal dystrophy, bullous keratopathy, keratoconus, corneal decompensation, corneal ulcer, neuroparalytic keratopathy, diabetic keratopathy, chemical burns of the cornea, or thermal burns of the cornea;
[12] The agent for use according to any one of [1] to [10] above, wherein the sensory neuropathy is caused by corneal surgery and the corneal surgery is keratoplasty;
[13] The agent for use according to any one of [1] to [10] above, wherein the sensory neuropathy is caused by corneal surgery and the corneal surgery is myopia correction surgery;
[14] The agent for use according to any one of [1] to [10] above, wherein the sensory neuropathy is caused by corneal surgery and the corneal surgery targets the cornea required for the treatment of eye disease or corneal injury;
[15] The agent for use according to any one of [1] to [14] above, wherein the sensory neuropathy caused by the corneal sensory nerve damaged due to corneal disease or corneal surgery is imperceptions;
[16] The agent for use according to any one of [1] to [14] above, wherein the sensory neuropathy caused by the corneal sensory nerve damaged due to corneal disease or corneal surgery is dry eyes;
[17] The agent for use according to any one of [1] to [16] above, which is in the form of an ophthalmic preparation.

### Advantageous Effects of Invention

According to the present invention, it became possible to treat or prevent sensory neuropathy caused by corneal disease or corneal surgery with the use of an agent comprising a xanthone compound having a semaphorin 3A inhibitory activity as an active ingredient. That is to say, according to the present invention, it became possible to treat or prevent sensory neuropathy caused by corneal disease, or sensory neuropathy caused by corneal surgery such as keratoplasty, myopia correction surgery or corneal surgery that targets the cornea required for the treatment of eye disease or corneal injury, and particularly, sensory neuropathy caused by the corneal sensory nerve damaged due to corneal disease or corneal surgery. In addition, it has been clarified that, according to the present invention, a xanthone compound can be effectively used as a promoter for the regeneration of the corneal sensory nerve, and it can also be used as a regeneration promoter for treating or preventing sensory neuropathy caused by the corneal sensory nerve damaged due to corneal disease or corneal surgery.

Moreover, the xanthone compound used in the present invention is chemically extremely stable in an aqueous solution such as a phosphate buffer. When the present xanthone compound is used for treatment or prevention of sensory neuropathy caused by corneal disease or corneal surgery, or as a regeneration promoter, it is most preferably used by eye drop administration. Accordingly, it has been clarified that the xanthone compound used in the present invention is extremely preferable because it is stable in tears or in the cornea after being administered in the form of an eye drop.

### Brief Description of Drawings

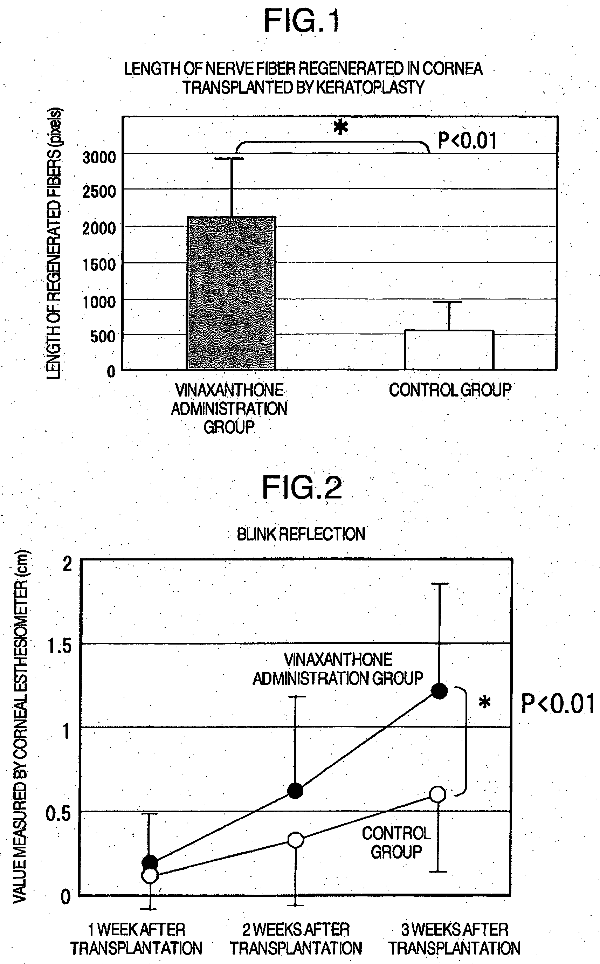

[Fig. 1] Fig. 1 is a view showing the results obtained by measuring the length of a nerve fiber regenerated in a cornea transplanted by keratoplasty in Example 1, namely, a corneal graft [Student's t-test, comparison with a control group, *: p < 0.01]. The longitudinal axis indicates a total of the lengths of the regenerated fibers. The horizontal axis indicates a vinaxanthone (SPF-3059-5) administration group and a control group (to which only a solvent containing no drugs was administered). From Fig. 1, it is found that the regeneration of nerve fibers in the corneal graft has been promoted by administration of vinaxanthone.
[Fig. 2] Fig. 2 is a view showing the results obtained by measuring corneal sensitivity in the central portion of a corneal graft using a Cochet-Bonnet corneal esthesiometer every week after completion of the surgery, in which this measurement was carried out to evaluate the function of the regenerated nerve in Example 1 [Mann-Whitney U test, comparison with a control group, *: p < 0.01]. The longitudinal axis indicates the level of corneal sensitivity. The horizontal axis indicates the results obtained 1 week, 2 weeks, and 3 weeks after completion of the transplantation. In Fig. 2, the filled circle (●) indicates a vinaxanthone administration group, and the open circle (○) indicates a control group to which only a solvent containing no drugs was administered. From Fig. 2, it is found that corneal sensitivity was improved by administration of vinaxanthone. As a result of the improvement of corneal sensitivity, it is found that the regenerated nerve in Example 1 was mainly the corneal sensory nerve.

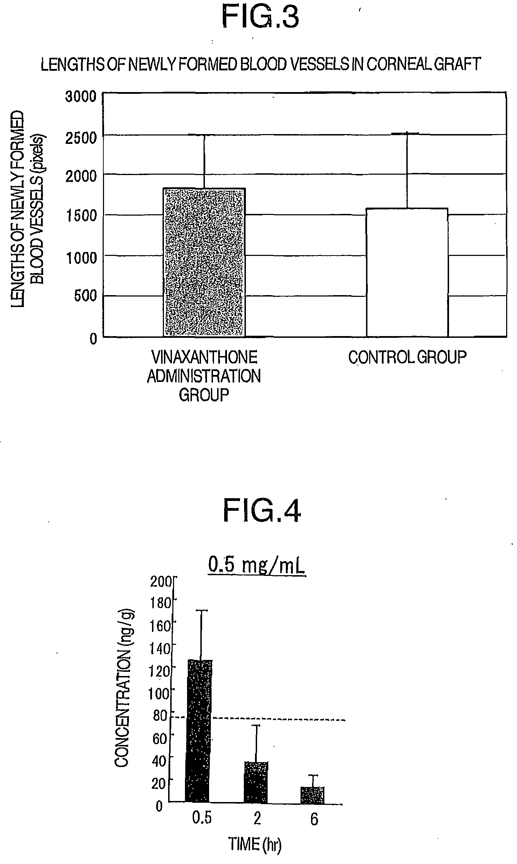

[Fig. 3] Fig. 3 is a view showing the results obtained by measuring the number of blood vessels that have been newly formed in the transplanted corneal graft in Example 1 [Student's t-test, no significant difference found in comparison with a control group]. The longitudinal axis indicates a total of lengths of the newly-formed blood vessels. The horizontal axis indicates a vinaxanthone (SPF-3059-5) administration group and a control group (to which only a solvent containing no drugs was administered). From Fig. 3, it is found that new formation of blood vessels into the corneal graft was not promoted by administration of vinaxanthone.
[Fig. 4] Fig. 4 is a view showing the results obtained by measuring the retention of vinaxanthone in the cornea after administration in the form of an eye drop, in which the concentration of the vinaxanthone solution was set at 0.5 mg/mL in Example 2. The dotted line indicates the IC50 value (75 ng/mL = 130 nM) obtained in the in vitro experiment. The longitudinal axis indicates the concentration of the retained vinaxanthone (ng/g). The number in the horizontal axis indicates the retention level of vinaxanthone after a certain period of time passed (e.g. the number 0.5 indicates the retention level 0.5 hours after the administration, the number 2 indicates the retention level 2 hours after the administration, and the number 6 indicates the retention level 6 hours after the administration). From Fig. 4, it is found that vinaxanthone that had been administered in a concentration of 0.5 mg/mL in the form of an eye drop was retained for 0.5 hours after the administration in a concentration in the cornea that was considered to be necessary for the expression of drug effects.

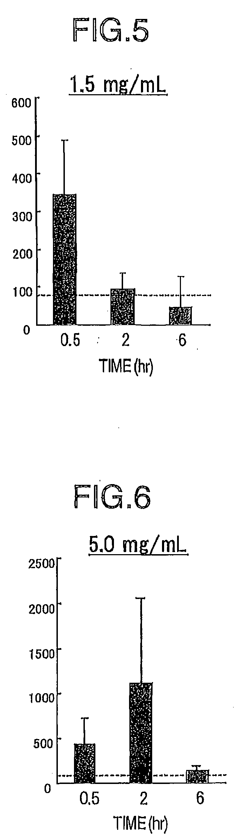

[Fig. 5] As with Fig. 4, Fig. 5 is a view showing the results obtained by measuring the retention of vinaxanthone in the cornea after administration in the form of an eye drop, in which the concentration of the vinaxanthone solution was set at 1.5 mg/mL in Example 2.
[Fig. 6] As with Fig. 4, Fig. 6 is a view showing the results obtained by measuring the retention of vinaxanthone in the cornea after administration in the form of an eye drop, in which the concentration of the vinaxanthone solution was set at 5.0 mg/mL in Example 2.

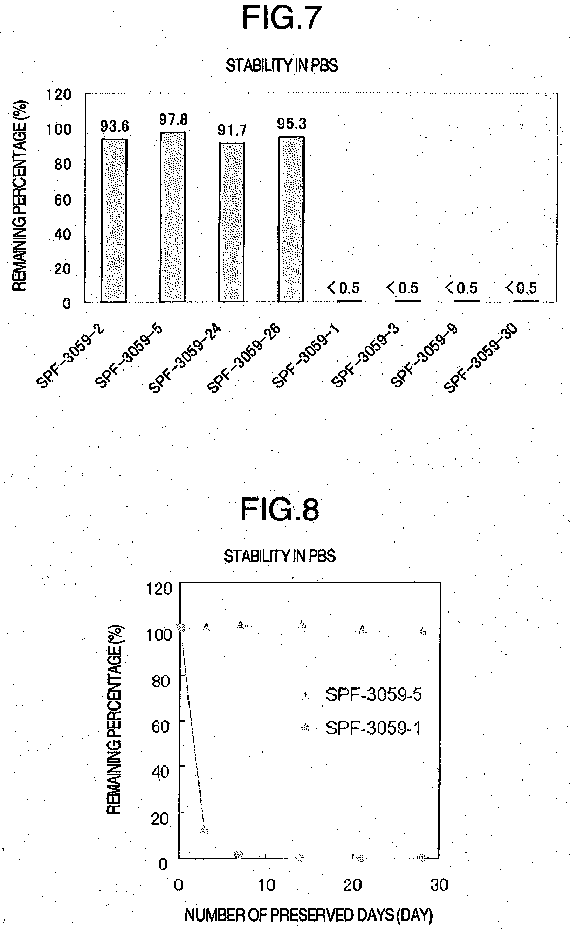

[Fig. 7] Fig. 7 is a view showing the results obtained by measuring the stability of a xanthone compound in PBS (phosphate buffer) in Example 10. The horizontal axis indicates the name of each compound used in the measurement. The longitudinal axis indicates the remaining percentage of each compound. From Fig. 7, it is found that all of the xanthone compounds excluded from the formula (1) were almost completely decomposed, whereas the xanthone compounds included in the formula (1) remained at a remaining percentage of 90% or more.
[Fig. 8] Fig. 8 is a view showing results obtained by measuring a change over time in the stability of vinaxanthone (SPF-3059-5) and SPF-3059-1 in PBS (phosphate buffer) in Example 11. The horizontal axis indicates the number of days in which each compound was conserved after dissolution. The longitudinal axis indicates the remaining percentage of each compound. In Fig. 8, the filled triangle (▲) indicates a change over time in the stability of vinaxanthone, and the filled circle (●) indicates a change over time in the stability of SPF-3059-1. From Fig. 8, it is found that SPF-3059-1 was almost completely decomposed for approximately 1 week, whereas vinaxanthone remained in a remaining percentage of 96% or more at 37°C in PBS for 4 weeks or longer, thereby showing stability for 4 weeks or longer.

### Description of Embodiments

In the present specification, the term "alkoxycarbonyl group" is used to mean a linear or branched alkoxycarbonyl group containing 2 to 7 carbon atoms. Specific examples of the alkoxycarbonyl group include a methoxycarbonyl group, an ethoxycarbonyl group, a propoxycarbonyl group, a 1-methylethoxycarbonyl group, a butoxycarbonyl group, a 1-methylpropoxycarbonyl group, a 2-methylpropoxycarbonyl group, a 1,1-dimethylethoxycarbonyl group, a pentyloxycarbonyl group, and a hexyloxycarbonyl group.

The term "acyloxy group" is used herein to mean a linear or branched acyloxy group containing 2 to 6 carbon atoms. Specific examples of the acyloxy group include an acetoxy group, a propionyloxy group, a butyryloxy group, an isobutyryloxy group, a valeryloxy group, an isovaleryloxy group, and a pivaloyloxy group.

The term "pharmaceutically acceptable salt" is used herein to mean a pharmaceutically or veterinarily acceptable salt. Examples of such a pharmaceutically acceptable salt include: inorganic basic salts such as a sodium salt, a potassium salt, a calcium salt, a magnesium salt, an aluminum salt, and an ammonium salt; organic basic salts such as a triethylammonium salt, a triethanolammonium salt, a pyridinium salt, and a diisopropylammonium salt; and basic amino acid salts such as arginine and lysine. In addition, when the compound represented by the formula (1) has two carboxyl groups for example, salts such as a monosodium monopotassium salt are also included in the present pharmaceutically acceptable salt.

Examples of the "corneal disease" in the phrase "sensory neuropathy caused by corneal disease" include keratitis, leukoma (due to corneal herpes, measles, syphilis, or injury), corneal infection, corneal degeneration, corneal dystrophy, corneal stromal dystrophy, bullous keratopathy, keratoconus, corneal decompensation, corneal ulcer, neuroparalytic keratopathy, diabetic keratopathy, chemical burns of the cornea (which is caused by a chemical or the like that has entered into the eyes), and thermal burns of the cornea.

Examples of the "corneal surgery" in the phrase "sensory neuropathy caused by corneal surgery" include keratoplasty, myopia correction surgery, and corneal surgery that targets the cornea required for the treatment of eye disease or corneal injury.

"Keratoplasty" is also referred to as corneal transplantation or corneal plasty. Examples of such keratoplasty include penetrating keratoplasty, lamellar keratoplasty, deep lamellar keratoplasty, sclerokeratoplasty, scleral graft transplantation, limbus corneae transplantation, and amnion transplantation. Examples of the purpose of performing keratoplasty include an optical purpose, a therapeutic purpose, a surgical purpose, and a cosmetic purpose. Specific examples of the keratoplasty include: keratoplasty performed to treat causative diseases such as keratitis, leukoma (due to corneal herpes, measles, syphilis, or injury), corneal infection, corneal degeneration, corneal dystrophy, corneal stromal dystrophy, bullous keratopathy (eyes subjected to intraocular lens implantation, Fuchs' corneal endothelial dystrophy, or non-crystal lens eyes), keratoconus, and corneal decompensation; keratoplasty performed to treat infections such as corneal ulcer (bacterial, fungal, viral, or sterile ulcer); keratoplasty performed to treat injury; keratoplasty performed to treat chemical burns of the cornea or thermal burns of the cornea; and keratoplasty performed to treat neurotrophic keratitis such as diabetic keratopathy.

Specific examples of the "myopia correction surgery" which is currently carried out include radial keratotomy (RK), photorefractive keratectomy (PRK), and laser in situ keratomileusis (LASIK). In addition, myopia correction methods, such as LASEK, intrastromal corneal rings, intracorneal lens, phakic intraocular lens, and orthokeratology, are also included in the myopia correction surgery.

Examples of the "eye disease" in the phrase "corneal surgery that targets the cornea required for the treatment of eye disease or corneal injury" include keratitis, corneal herpes, keratoconus, corneal degeneration, leukoma, bullous keratopathy, keratomalacia, and cataract.

Examples of the "corneal injury" in the phrase "corneal surgery that targets the cornea required for the treatment of eye disease or corneal injury" include injuries caused by an irritant liquid entering into the eyes, a solid flying and entering into the eyes, cutting with a knife, sticking with a knife or the like, scratching with a pet animal, an improper use of contact lens, strong beam, and contusion.

Examples of the "corneal surgery" in the phrase "corneal surgery that targets the cornea required for the treatment of eye disease or corneal injury" include keratotomy, keratectomy, keratorefractive surgery, orthokeratology, and keratomileusis. In addition, in a case in which the eye disease is cataract, the surgical operation that targets the cornea required for the treatment of cataract includes an operation for insertion of artificial lens (artificial crystalline lens).

Examples of the "sensory neuropathy" in the phrase "sensory neuropathy caused by corneal disease or corneal surgery" include reduction in tactile sense and pain sense. The lack of blink reflection is caused by imperception, and it may result in dry eyes, injured eyeballs, etc. Accordingly, the "sensory neuropathy" also includes dry eyes. It is to be noted that the term "sensory nerve" means sensory nerve distributed in the cornea, and thus, it has the same meaning as the trigeminal nerve traveling in the cornea (the ophthalmic nerve traveling in the cornea), namely, the corneal sensory nerve.

The term "promoter for the regeneration of the corneal sensory nerve" is used to mean a drug having an action to promote the regeneration of the corneal sensory nerve. The term "action to promote the regeneration of the corneal sensory nerve" is used to mean an action to promote the regeneration of the corneal sensory nerve that has been disconnected or damaged by corneal surgery or the like.

In the compound represented by the formula (1), R¹ and R³ each independently represent a hydrogen atom, a carboxyl group, or an alkoxycarbonyl group. Examples of a preferred alkoxycarbonyl group include alkoxycarbonyl groups containing 2 to 4 carbon atoms, such as a methoxycarbonyl group, an ethoxycarbonyl group, or a propoxycarbonyl group. Among them, a methoxycarbonyl group is more preferable, As R¹, a hydrogen atom or a carboxyl group is preferable, and of these, a carboxyl group is more preferable. As R³, a hydrogen atom or a carboxyl group is preferable, and of these, a carboxyl group is more preferable.

R² and R⁴ each independently represent a hydrogen atom, a hydroxyl group, or an acyloxy group. Examples of a preferred acyloxy group include acyloxy groups containing 2 to 4 carbon atoms, such as an acetoxy group, a propionyloxy group, a butyryloxy group, or an isobutyryloxy group. Among them, an acetoxy group is more preferable. As R², a hydrogen atom or a hydroxyl group is preferable, and of these, a hydroxyl group is more preferable. As R⁴, a hydrogen atom or a hydroxyl group is preferable, and of these, a hydroxyl group is more preferable.

Specific examples of the compound represented by the formula (1) include: SPF-3059-2 in which R¹ is a carboxyl group, R² is a hydroxyl group, R³ is a hydrogen atom, and R⁴ is a hydroxyl group; SPF-3059-4 in which R¹ is a carboxyl group, R² is a hydroxyl group, R³ is a carboxyl group, and R⁴ is a hydrogen atom; SPF-3059-5 in which R¹ is a carboxyl group, R² is a hydroxyl group, R³ is a carboxyl group, and R⁴ is a hydroxyl group; SPF-3059-12 in which R¹ is a carboxyl group, R² is a hydrogen atom, R³ is a carboxyl group, and R⁴ is a hydroxyl group; SPF-3059-24 in which R¹ is a hydrogen atom, R² is a hydroxyl group, R³ is a carboxyl group, and R⁴ is a hydroxyl group; SPF-3059-25 in which R¹ is a hydrogen atom, R² is a hydroxyl group, R³ is a carboxyl group, and R⁴ is a hydrogen atom; and SPF-3059-26 in which R¹ is a hydrogen atom, R² is a hydroxyl group, R³ is a hydrogen atom, and R⁴ is a hydroxyl group. Among these compounds, SPF-3059-5 is most preferable. SPF-3059-5 is also referred to as vinaxanthone.

As a pharmaceutically acceptable salt of the compound represented by the formula (1), a sodium salt, a potassium salt, and a calcium salt are preferable because these salts have an improved solubility in water and the pH of the solution does not need to be adjusted. Among these salts, a sodium salt is most preferable.

The compound represented by the formula (1) can be obtained by the culture or chemical total synthesis of Penicillium sp. SPF-3059 strain, or by chemical conversion according to a known synthetic method using a product obtained by the aforementioned culture or total synthesis as a raw material.

That is to say, in the case of applying a culture method, the compound represented by the formula (1) can be effectively obtained by culturing a mold belonging to Penicillium sp. isolated from the earth in Osaka prefecture, that is, a SPF-3059 strain [wherein this strain was deposited under the terms of the Budapest Treaty on the International Recognition of the Deposit of Microorganisms for the Purposes of Patent Procedure, with the NITE Patent Microorganisms Depositary, the National Institute of Advanced Industrial Science and Technology, an Independent Administrative Institution under the Ministry of Economy, Trade and Industry (the AISTTsukuba Central 6, Higashi 1-1-1, Tsukuba, Ibaraki, Japan, postal code: 305-8566), under accession No. FERM BP-7663 on July 13, 2001]. Specifically, the compound represented by the formula (1) can be obtained in accordance with the method described in International Publication WO02/09756 (the above-mentioned Patent Literature 1) or International Publication WO03/062243 (the above-mentioned Patent Literature 2).

In the case of applying total synthesis, the compound represented by the formula (1) can be obtained according to the method described in JP 2008-13530 A.

Among the compounds represented by the formula (1), a compound in which at least one of R¹ and R³ represents an alkoxycarbonyl group or at least one of R² and R⁴ represents an acyloxy group can be synthesized by performing known esterification on the compound represented by the formula (1) in which the concerned alkoxycarbonyl group is a carboxy group, used as a raw material, and/or by performing known acylation on the compound represented by the formula (1) in which the concerned acyloxy group is a hydroxyl group, used as a raw material, thereby performing chemical conversion of the compound. Such known esterification or acylation may be carried out according to the method described in JP 2006-335683 A or International Publication WO03/062440 (the above-mentioned Patent Literature 3).

Moreover, a pharmaceutically acceptable salt of the compound represented by the formula (1) can be obtained by allowing a base to act on the compound represented by the formula (1) obtained by any one of the above described methods in a suitable solvent such as water, methanol, ethanol, acetone, ethyl acetate, chloroform, or ether.

The compound represented by the formula (1) of the present invention is also referred to as a xanthone compound. As it will have become apparent in Examples 4 and 5 later, this is a compound having an action to inhibit the neurite elongation activity of semaphorin 3A. As it will have become apparent in Example 2 later, it has been elucidated for the first time by the present invention that the xanthone compound represented by the formula (1) promotes the regeneration of the corneal sensory nerve in a mouse keratoplasty model. Accordingly, the present xanthone compound is effective for the treatment or prevention of corneal sensory neuropathy caused by the corneal sensory nerve damaged due to corneal disease or corneal surgery. An example of the sensory neuropathy caused by the corneal sensory nerve damaged due to corneal disease or corneal surgery is reduction in tactile sense or pain sense. The lack of blink reflection is caused by such imperception, and it then results in dry eyes, damage on eyeballs, etc. Accordingly, the xanthone compound represented by the formula (1) is effective particularly for the prevention or treatment of reduction in tactile sense, pain sense or the like, or dry eyes.

As it will have become apparent in Example 1 later, the xanthone compound represented by the formula (1) promotes the regeneration of the corneal sensory nerve, and thus, it is also effective as a promoter for the regeneration of the corneal sensory nerve. By promoting the regeneration of the corneal sensory nerve, sensory neuropathy caused by the corneal sensory nerve damaged due to corneal disease or corneal surgery can be treated or prevented. More specifically, the xanthone compound represented by the formula (1) is effective for preventing or treating imperception and dry eyes caused by the corneal sensory nerve damaged due to corneal disease or corneal surgery.

The agent for use in treatment or prevention of corneal sensory neuropathy or for promoting regeneration of corneal sensory nerve according to the present invention comprises, as an active ingredient, the compound represented by the formula (1) or a pharmaceutically acceptable salt thereof. In addition, additive components used for various types of preparations, such as a pharmaceutically acceptable common carrier, binder, stabilizer, excipient, diluent, pH adjuster, disintegrator, solubilizer, solubilizing agent, and isotonizing agent, may be added to the present therapeutic or preventive agent or the present regeneration promoter.

Agents for use according to the invention are parenterally administered topically into eyes. For parenteral administration, the agents can be processed into a dosage form such as ophthalmic preparations, ophthalmic ointments, intraocular injections, subconjunctival injections, intravenous injection preparations (drops), intramuscular injections, hypodermic injections, or nasal preparations (sprays for intranasal administration). Among others, ophthalmic preparations are preferable. In the case of liquid preparations, the agents can be processed into solutions, emulsions, suspensions or the like, as appropriate. Among others, the solution for ophthalmic preparations using a phosphate buffer is preferable. For parenteral administration, an active ingredient may be dissolved or suspended in a physiologically acceptable carrier such as water, a normal saline, oil, a glucose aqueous solution, etc., and the thus obtained solution or suspension may comprise an auxiliary agent such as an emulsifier, a stabilizer, an osmoregulatory salt or a buffer, as necessary. In the case of ophthalmic preparations, it may comprise additives including isotonizing agents such as glycerin or sodium chloride, buffers such as phosphoric acid or citric acid, pH adjusters such as hydrochloric acid or sodium hydroxide, thickeners such as hydroxypropylmethyl cellulose or polyvinyl alcohol, preservatives such as benzethonium chloride, or solubilizers, as necessary. In addition, examples of additives for an eye ointment include petrolatum, polyethylene glycol, purified lanolin, and liquid paraffin.

The applied dose and the frequency of administration are different depending on an administration method, the age, body weight and symptoms of a patient, and the like. The agent is preferably administered once or twice or more times per day. When the agent is administered twice or more times per day, it is desired to repeatedly administer the agent every day or at appropriate intervals. Since the regeneration of the corneal sensory nerve generally requires a period of time, ranging from several days to several months, it is desired to continuously administer the agent to suppress the activity of semaphorin for the aforementioned period of time.

With regard to the applied dose, the agent can be administered at a dose of several hundreds of µg to 2 g, preferably 5 to 100 mg, and more preferably several tens of mg or less, to an adult patient, relative to the amount of an active ingredient for a single administration. The agent can be administered once or divided over several administrations per day. In order to reduce the frequency of administration, a sustained-release preparation can also be used. It is also possible to administer the agent in small amounts over a long period of time using an osmotic pump or the like. In the case of parenteral administration, the agent can be administered at a dose of 0.1 to 100 mg/day, and more preferably 0.3 to 50 mg/day, to an adult patient. The agent can be administered once or divided over several administrations per day. In order to reduce the frequency of administration, a sustained-release preparation can also be used.

In a case in which the agent is used in the form of an eye drop, the agent can be administered at a dose of 0.01 to 10 w/v%, and preferably 0.05 to 5 w/v%, to an adult patient, relative to the amount of an active ingredient. It is desired to administer the agent in an amount of one to several droplets for a single administration one to six times per day, depending on symptoms. As it will have become apparent in Example 2 later, the xanthone compound represented by the formula (1) is excellent in retention in the cornea when administered as ophthalmic preparations. Accordingly, the preferred dosage form of the present xanthone compound is ophthalmic preparations. In addition, when the present xanthone compound is used in the form of ophthalmic ointments, it can be administered at a dose of 0.01 to 10 w/w%, and preferably 0.1 to 5 w/w%, relative to the amount of an active ingredient thereof Such an eye ointment is desirably administered one to six times per day, depending on symptoms.

In all of these administration methods, it is preferable to adopt an administration route and an administration method, which achieve the concentration of the compound that is sufficient for inhibiting the activity of semaphorin in its site of action. Furthermore, the therapeutic or preventive agent for sensory neuropathy caused by corneal disease or corneal surgery and the promoter for the regeneration of the corneal sensory nerve according to the present invention can also be used as agents for animals. Among such animals, mammals are preferable, and a human is most preferable.

Hereinafter, the present invention will be specified more in detail in the following examples. However, these examples are not intended to limit the technical scope of the present invention.

### Example 1

### Action of vinaxanthone to promote regeneration of corneal sensory nerve in corneal graft in mouse keratoplasty model

In this experiment, there were used genetically modified mice (P0-Cre/Floxed-EGFP mouse), in which corneal parenchymal cells, corneal endothelial cells, and nerves expressed a type of fluorescent protein, Green Fluorescent Protein (GFP), in the cornea. Using these mice, corneal sensory nerve fibers traveling in the cornea can easily be observed by removing a layer of corneal endothelial cells.

The cornea derived from a syngeneic wild-type mouse was transplanted into each of the above-mentioned mice. Thereafter, 50 ul of vinaxanthone (which had been dissolved in Rinderon (1 mg/ml betamethasone sodium phosphate injection solution) to a concentration of 0.1 mg/mL) was administered to the mice via subconjunctival injection immediately after completion of the operation and then, every two days in a total of 11 times. Only a solvent containing no drugs was administered in the same amount as described above to a control group. The removal of the suture was conducted one week after completion of the operation. Three weeks after completion of the operation, corneal sensitivity was evaluated, the mice were then subjected to euthanasia, and eyeballs were then excised. A comparison was made regarding the regeneration of nerve fibers in the corneal graft. Corneal sensitivity was evaluated by measuring corneal sensitivity in the center of the corneal graft every week after completion of the operation, using a Cochet-Bonnet corneal esthesiometer. The comparison regarding the regeneration of nerve fibers in the corneal graft was carried out by: determining fibers in the corneal graft that were double-positive to β3 tubulin and GFP as regenerated nerve fibers according to an immunostaining method using a β3 tubulin antibody; tracing the regenerated nerve fibers using image processing software of a computer; measuring a total of the lengths thereof, and then making a comparison between the vinaxanthone administration group and the control group.

From the results shown in Fig. 1, it was found that the regeneration of nerve fibers in the corneal graft was promoted in the vinaxanthone administration group more significantly than in the control group.

In addition, from the results shown in Fig. 2, it was found that the improvement of blink reflection was observed in the vinaxanthone administration group more significantly than in the control group three weeks after completion of the operation, so that corneal sensitivity could be improved in the vinaxanthone administration group. These results suggested that the regenerated nerves shown in Fig. 1 be mainly corneal sensory nerves.

With regard to corneal vascularization, fibers that exhibited positive in an immunostaining method using a CD31 antibody were defined as newly formed blood vessels, and the newly formed blood vessels were traced with image processing software of a computer. Thereafter, a total of the lengths thereof were measured, and a comparison was then made between the vinaxanthone administration group and the control group.

From the results shown in Fig. 3, it was found that vascularization into the corneal graft was not promoted in the vinaxanthone administration group, in comparison with the control group.

These results demonstrated that vinaxanthone promotes the regeneration of the disconnected corneal sensory nerve, and also promotes the recovery of the nerve function, namely, corneal sensitivity. Also, it became clear that vinaxanthone does not promote corneal vascularization, which is a harmful phenomenon.

### Example 2

### Evaluation of retention of vinaxanthone preparation in corneal tissues

Rabbits (Kbs: JW, healthy, male, body weight: 2.00 to 2.49 kg) were each administered with 50 µL of a PBS solution (0.12 M phosphate buffer (pH 7.4)) of vinaxanthone (0.5, 1.5 and 5.0 mg/mL) via eye drops administration onto the cornea of the right eye, and their eyes were then closed for 30 seconds. Thereafter, 0.5, 2, and 6 hours after completion of the administration, the rabbits were subjected to euthanasia, and the excised eyeballs were then washed with a normal saline. Thereafter, the cornea was collected from each eyeball. The cornea was homogenized, and vinaxanthone was then extracted therefrom. A change over time in the content and concentration of vinaxanthone in the corneal tissues was examined using HPLC.

From the results shown in Figs. 4, 5, and 6, it was confirmed that vinaxanthone was transferred into the corneal tissues in an applied concentration/dose dependent manner. In the 5.0 mg/mL vinaxanthone solution administration group, it was confirmed that vinaxanthone was retained in the cornea in an amount larger than the IC₅₀ value (75 ng/mL = 130 nM, shown with the dotted line in each figure) obtained by an in vitro experiment (an experiment regarding inhibitory activity on the collapse activity of Sema3A in Example 4) even 6 hours after completion of the administration. It became clear that the concentration of vinaxanthone in the cornea, which is considered to be necessary for the expression of drug effects, can be achieved even by eye drops administration.

As a result, it was suggested that eye drops administration is a realistic method of administering vinaxanthone.

### Example 3

### Production of xanthone compound represented by formula (1)

The xanthone compounds represented by the formula (1) of the present invention are all known compounds, and are disclosed in International Publication WO02/09756 (the above-mentioned Patent Literature 1), International Publication WO03/062243 (the above-mentioned Patent Literature 2), International Publication WO03/062440 (the above-mentioned Patent Literature 3), JP 2006-335683 A, and JP 2008-13530 A. The present xanthone compounds can be produced by the culture, chemical total synthesis, or chemical conversion of the SPF-3059 strain. In addition to production methods, the physicochemical properties of the compounds are also described in the aforementioned patent literatures. The production method and the like are specifically as follows.

10 ml of a medium containing 2% glucose, 5% sucrose, 2% cottonseed powder, 0.1% sodium nitrate, 0.1% L-histidine, 0.05% dipotassium phosphate, 0.07% potassium chloride and 0.0014% magnesium sulfate heptahydrate, which had been adjusted to be pH 7.0, was poured into a 50 ml volume Erlenmeyer flask, and it was then sterilized in an autoclave. To the resulting medium, a platinum loop of the slant-cultured Penicillium sp. SPF-3059 strain (FERM BP-7663) was inoculated, and the obtained mixture was then subjected to a rotary stirring culture at 27°C at 180 rpm for 4 days to obtain a pre-pre-culture. 125 ml of a medium having the same composition as described above was poured into each of five 500 ml volume Erlenmeyer flasks, and it was then sterilized in an autoclave. 4 ml of the pre-pre culture medium was added to each of the resulting media, and it was then subjected to a rotary stirring culture at 27°C at 180 rpm for 4 days to obtain a pre-culture. 30 L of a medium containing 1.43% glucose, 3.57% sucrose, 1.43% cottonseed powder, 0.07% sodium sulfate, 0.07% L-histidine, 0.036% dipotassium phosphate, 0.05% potassium chloride, 0.001% magnesium sulfate heptahydrate and 0.01% Adekanol LG-295S (a defoaming agent manufactured by ADEKA Corporation), which had been adjusted to be pH 7.0, was poured into a 50 L volume jar fermenter, and it was then subjected to high-pressure steam sterilization (121°C, 20 minutes). Thereafter, 500 ml of the above described pre-culture medium was added to the resulting medium, and the obtained mixture was then subjected to a ventilation stirring culture at 27°C at 400 rpm at a ventilation volume of 15L/min for 9 days.

After completion of the culture, the culture medium was centrifuged at 10,000 rpm for 10 minutes to separate a supernatant from a cell mass. The supernatant fraction was extracted twice with 20 L of ethyl acetate-formic acid (99:1). The cell mass fraction was extracted with 30 L of acetone, and it was then filtrated and concentrated. The resulting aqueous solution was extracted with 10 L of ethyl acetate-formic acid (99:1). The two above extracts were mixed with each other, and the obtained mixture was then subjected to vacuum concentration to obtain 224 g of a crude extract. 100 g of the crude extract was dissolved in 500 ml of methanol. The obtained solution was then subjected to column chromatography using Sephadex (registered trademark) LH-20 (GE Healthcare), and was then eluted with methanol. Active fractions were gathered, and the solvent was then distilled away under a reduced pressure to obtain 48.8 g of an oily product. The obtained oily product was dissolved in 400 ml of methanol. The resultant was then subjected to column chromatography using TSKgel TOYOPERL HW-40F (Tosoh Corporation), and was then eluted with methanol. Active fractions were gathered, and the solvent was then distilled away under a reduced pressure to obtain 21.8 g of a roughly purified product. This product was dissolved by 200 mg each time in 2 ml of dimethyl sulfoxide (DMSO), and the obtained solution was then subjected to reversed-phase separatory HPLC. Conditions for the reversed-phase separatory HPLC were the following. Column: Wakopak-Wakosil (registered trademark)-II5C18HGprep (5 cm in diameter x 10 cm connected with 5 cm in diameter x 25 cm, manufactured by Wako Pure Chemical Industries Ltd.), eluent A: 1% formic acid aqueous solution, eluent B: methanol, gradient: a linear gradient in which the percentage of solution B was 45% → 75% for 2 hours, flow rate: 25 ml/min, and detection: absorbance at 260 nm. The eluent was separated every minute.

The thus separated fractions were analyzed by analytical HPLC. Conditions for the analytical HPLC were the following. Column: Wakopak-Wakosil (registered trademark)-II5C18RS (4.6 mm in diameter x 150 mm, manufactured by Wako Pure Chemical Industries Ltd.), eluent A: 1% formic acid aqueous solution, eluent B: methanol, gradient: a linear gradient in which the percentage of solution B was 20% → 67% for 71.1 minutes, flow rate: 1.3 ml/min, and detection: absorbance at 260 nm. Using the retention time in this analytical HPLC as an indicator, the separatory HPLC elution fractions were gathered, and the solvent was then distilled away under a reduced pressure. The residue was subjected to separatory HPLC again, and purification was then carried out in the same manner as described above. The resultant was further subjected to column chromatography using TSKgel TOYOPERLHW-40F (Tosoh Corporation), and purification was then carried out in the same manner as described above. The solvent was distilled away under a reduced pressure, and the residue was then dried to obtain a purified product of the desired compound.

The specifically obtained compounds and their physicochemical properties are as follows.

### SPF-3059-2

- Appearance: cream-colored powder
- High-resolution high-speed electron bombardment mass spectrum (HRFAB-MS) m/z (M+H)⁺: Actual measurement value: 533.0710, Calculation value: 533.0721
- Molecular formula: C₂₇H₁₆O₁₂
- Ultraviolet-visible absorption spectrum λmax (in methanol) nm (ε): 209 (40,600), 236 (42,600), 283 (28,500), 323 (25,400)
- Infrared absorption spectrum vmax (KBr)cm⁻¹: 3266, 1678, 1654, 1623, 1562, 1471, 1296
- ¹H-NMR (DMSO-d₆) δ ppm: 2.53 (6H, s), 6.93 (1H, s), 6.95 (1H, s), 7.47 (1H, s), 8.15 (1H, s), 8.54 (1H, s), 9.38 (1H, brs), 9.89 (1H, brs), 10.78 (1H, brs), 11.37 (1H, brs), 12.68 (1H, brs)
- ¹³C-NMR(DMSO-d₆) δ ppm: 29.1, 32.1, 102.3, 103.1, 108.7, 112.5, 113.5, 119.6, 119.8, 120.9, 126.2, 132.4, 133.6, 136.1, 141.7, 144.5, 150.71,150.74, 152.49, 152.54, 152.7, 154.4, 167.4, 172.9, 173.4, 199.2, 201.2

Based on the above data, the structural formula of the compound SPF-3059-2 was determined to be the following formula (2).

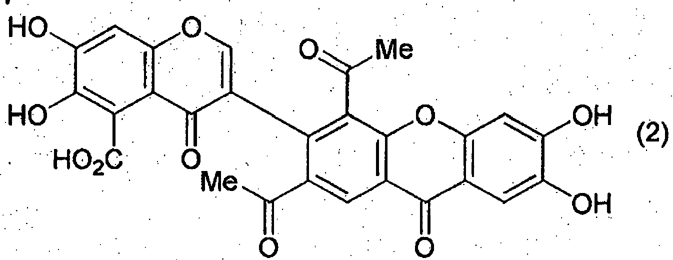

### SPF-3059-4

- Appearance: cream-colored powder
- Molecular weight: 560
- Molecular formula: C₂₈H₁₆O₁₃
- High-speed electron bombardment mass spectrum (FAB-MS) m/z (positive): 561 (M+H)⁺
- High-speed electron bombardment mass spectrum (FAB-MS) m/z (negative): 559 (M-H)⁻
- High-resolution high-speed electron bombardment mass spectrum (HRFAB-MS) m/z (M+H)⁺: Actual measurement value: 561.0667, Calculation value: 561.0670 (C₂₈H₁₇O₁₃)
- Ultraviolet-visible absorption spectrum λmax (in methanol) nm (e): 221 (35,600), 250 (38,100), 276sh (25,800), 323 (24,300)
- Infrared absorption spectrum vmax (KBr) cm⁻¹: 3412, 1665, 1619, 1563, 1465, 1427, 1263
- ¹H-NMR (DMSO-d₆) δ ppm: 2.53 (3H, s), 2.56 (3H, s), 6.84 (1H, d, 2.1), 6.95 (1H, s), 6.96 (1H, d, 2.1), 8.17 (1H, s), 8.52 (1H, s), 10.10-11.40 (3H, brs), 12.71 (1H, brs), 13.26 (1H, brs)
- ¹³C-NMR(DMSO-d₆) δ ppm: 29.2, 32.1, 102.3, 103.2, 110.1, 112.4, 112.8, 119.6, 120.3, 120.8, 126.3, 133.1, 133.4, 136.7, 137.5, 141.7, 150.8, 152.3, 152.7, 152.8, 157.2, 163.9, 167.4, 69.3, 172.2, 172.9, 199.3,201.0
- Solubility: Insoluble in water and hexane, soluble in methanol and DMSO

Based on the above data, the structural formula of the compound SPF-3059-4 was determined to be the following formula (3).

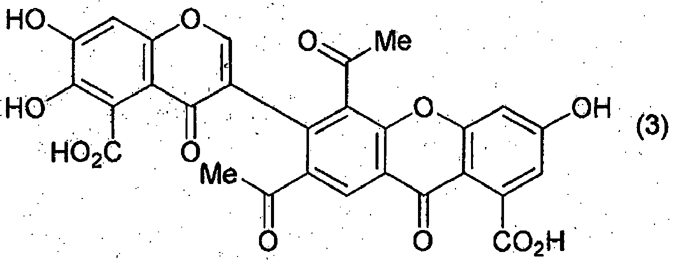

### SPF-3059-5 (vinaxanthone)

- Appearance: cream-colored powder
- High-resolution high-speed electron bombardment mass spectrum (HRFAB-MS) m/z (M+H)⁺: Actual measurement value: 577.0615, Calculation value: 577,0619
- Molecular formula: C₂₈H₁₆O₁₄
- Ultraviolet-visible absorption spectrum λₘₐₓ (in methanol) nm (ε): 229 (35,800), 284 (22,600), 322 (21,000)
- Infrared absorption spectrum vmax (KBr) cm⁻¹: 3260, 1684, 1626, 1567, 1467, 1288
- ¹H-NMR (DMSO-d₆) δ ppm: 2.53 (3H, s), 2.55 (3H, s), 6.93 (1H, s), 6.96 (1H, s), 8.17 (1H, s), 8.53 (1H, s), 9.5-13.0 (6H)
- ¹³C-NMR(DMSO-d₆) δ ppm: 29.1, 32.1, 102.26, 102.32, 109.9, 112.4, 119.6, 119.8, 120.3, 120.9, 126.3, 132.5, 133.4, 136.2, 141.2, 141.7, 150.4, 150.8, 152.1, 152.68, 152.73, 154.5, 167.4, 167.5, 172.5, 172.9, 199.1,201.1,

Based on the above data, the structural formula of the compound SPF-3059-5 (vinaxanthone) was determined to be the following formula (4).

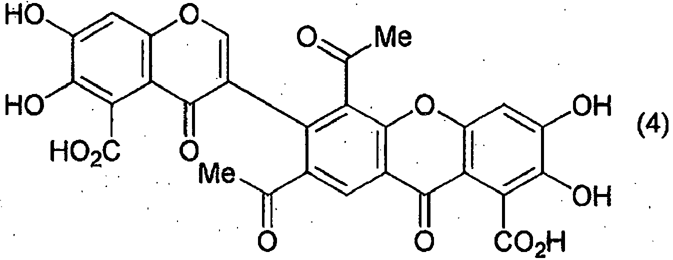

### SPF-3059-12

- Appearance: cream-colored powder
- Molecular weight: 560
- Molecular formula: C₂₈H₁₆O₁₃
- High-speed electron bombardment mass spectrum (FAB-MS) m/z (positive): 561 (M+H)⁺
- High-speed electron bombardment mass spectrum (FAB-MS) m/z (negative): 559 (M-H)⁻
- High-resolution high-speed electron bombardment mass spectrum (HRFAB-MS) m/z (M+H)⁺: Actual measurement value: 561.0680, Calculation value: 561.0670 (C₂₈H₁₇O₁₃)
- Ultraviolet-visible absorption spectrum λmax (in methanol) nm (ε): 232 (37,400), 250sh (34,800), 285 (28,000), 308sh (23,200), 360sh (9,000)
- Infrared absorption spectrum vmax (KBr) cm⁻¹: 3080, 1698, 1608, 1468, 1291
- ¹H-NMR (DMSO-d₆) δ ppm: 2.54 (3H, s), 2.55 (3H, s), 6.82 (1H, d, 2.1), 6.87 (1H, s), 6.95 (1H, d, 2.1), 8.22 (1H, s), 8.55 (1H, s), 9.50-13.50 (5H, brs)
- ¹³C-NMR (DMSO-d₆) δ ppm: 29.1, 32.2, 102.1, 103.0, 109.4, 112.1, 113.5, 119.8, 120.0, 121.7, 126.6, 132.0, 133.3, 135.9, 136.7, 141.7, 150.6, 152.1, 153.0, 155.4, 157.6, 162.4, 167.4, 167.6, 172.2, 172.9, 199.1, 201.1
- Solubility: Insoluble in water and hexane, soluble in methanol and DMSO

Based on the above data, the structural formula of the compound SPF-3059-12 was determined to be the following formula (5).

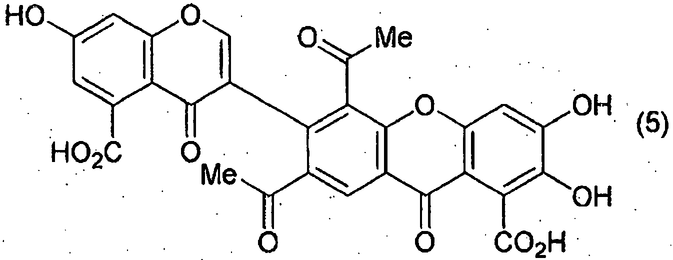

### SPF-3059-24

- Appearance: cream-colored powder
- Molecular weight: 532
- Molecular formula: C₂₇H₁₆O₁₂
- High-speed electron bombardment mass spectrum (FAB-MS) m/z (positive): 533 (M+H)⁺
- High-speed electron bombardment mass spectrum (FAB-MS) m/z (negative): 531 (M-H)⁻
- High-resolution high-speed electron bombardment mass spectrum (HRFAB-MS) m/z (M+H)⁺: Actual measurement value: 531.0621, Calculation value: 531.0564 (C₂₇H₁₇O₁₂)
- Ultraviolet-visible absorption spectrum λmax (in methanol) nm (ε): 212 (36,900), 229sh (34,500), 283 (26,300), 323 (21,700)
- Infrared absorption spectrum vmax (KBr) cm⁻¹: 3447, 1697, 1629, 1578, 1470, 1290
- ¹H-NMR (DMSO-d₆) δ ppm: 2.52 (3H, s), 2.54 (3H, s), 6.92 (1H, s), 6.93 (1H, s), 7.28 (1H, s), 8.13 (1H, s), 8.54 (1H, s), 9.50-13.00 (5H, brs)
- ¹³C-NMR (DMSO-d6) δ ppm: 29.1, 32.3, 102.3, 102.9, 107.9, 110.0, 115.8, 119.8, 120.4, 120.7, 126.5, 133.0, 133.3, 136.0, 141.2, 145.0, 150.4, 151.1, 152.2, 152.9, 153.0, 154.3, 167, 172.6, 173.6, 199.1, 201.1
- Solubility: Insoluble in water and hexane, soluble in methanol and DMSO

Based on the above data, the structural formula of the compound SPF-3059-24 was determined to be the following formula (6).

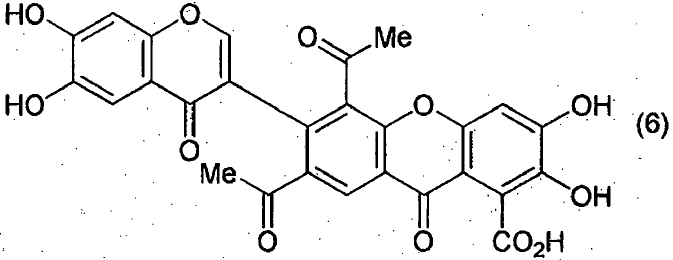

### SPF-3059-25

- Appearance: cream-colored powder
- Molecular formula: C₂₇H₁₆O₁₁
- High-speed electron bombardment mass spectrum (FAB-MS) m/z (positive): 517 (M+H)⁺
- High-speed electron bombardment mass spectrum (FAB-MS) m/z (negative): 515 (M-H)⁻
- High-resolution high-speed electron bombardment mass spectrum (HRFAB-MS) m/z (M+H)⁺: Actual measurement value: 517.0778, Calculation value: 517.0771 (C₂₇H₁₇O₁₁)
- Ultraviolet-visible absorption spectrum λmax (in methanol) nm (ε): 215 (35,000), 253 (35,100), 276sh (25,200), 323 (23,400)
- Infrared absorption spectrum vmax (KBr) cm⁻¹: 3417, 1691, 1625, 1471, 1293
- ¹H-NMR (DMSO-d₆) δ ppm: 2.54 (6H, s), 6.82 (1H, brs), 6.92 (2H, brs), 7.27 (1H, s), 8.14 (1H, s), 8.53 (1H, s), 9.5-14.0 (4H, brs)
- ¹³C-NMR (DMSO-d₆) δ ppm: 29.2, 32.3, 102.9, 103.0, 107.8, 109.9, 113.0, 115.7, 120.4, 120.6, 126.4, 133.3, 133.4, 136.4 (2c), 145.0, 151.2, 152.3, 152.98, 153.01, 157.3, 164.2, 169.4, 172.6, 173.6, 199.2, 201.0
- Solubility: Insoluble in water and hexane, soluble in methanol and DMSO

Based on the above data, the structural formula of the compound SPF-3059-25 was determined to be the following formula (7).

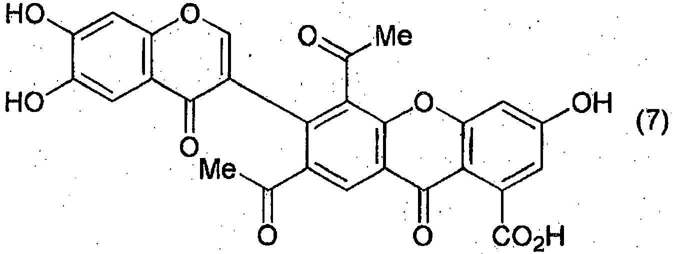

### SPF-3059-26

- Appearance: cream-colored powder
- Molecular weight: 488
- Molecular formula: C₂₆H₁₆O₁₀
- High-speed electron bombardment mass spectrum (FAB-MS) m/z (positive): 489 (M+H)⁺
- High-speed electron bombardment mass spectrum (FAB-MS) m/z (negative): 487 (M-H)⁻
- High-resolution high-speed electron bombardment mass spectrum (HRFAB-MS) m/z (M+H)⁺: Actual measurement value: 489.0823, Calculation value: 489.0822 (C₂₆H₁₇O₁₀)
- Ultraviolet-visible absorption spectrum λmax (in methanol) nm (ε): 212 (31,500), 235 (30,900), 284 (23,900), 324 (19,500)
- Infrared absorption spectrum vmax (KBr) cm⁻¹: 3454, 1694, 1625, 1517, 1471, 1293
- ¹H-NMR (DMSO-d₆) δ ppm: 2.53 (3H, s), 2.54 (3H, s), 6.91 (1H, s), 6.92 (1H, s), 7.27 (1H, s), 7.47 (1H, s), 8.11 (1H, s), 8.57 (1H, s)
- ¹³C-NMR (DMSO-d₆) δ ppm: 29.1,32.2, 102.9, 103.0, 107.9, 108.5, 113.3, 115.7, 119.8, 120.7, 126.3, 132.7, 133.5, 135.8, 144.6, 145.0, 150.8, 151.1, 152.5, 152.9 (2c), 154.7, 173.3, 173.6, 199.1, 201.2
- Solubility: Insoluble in water and hexane, soluble in methanol and DMSO

Based on the above data, the structural formula of the compound SPF-3059-26 was determined to be the following formula (8).

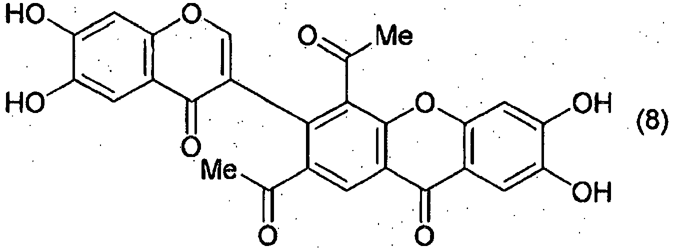

### Example 4

### Inhibitory activity of xanthone compound on collapse activity of Sema3A

A polylysine-coated 96-well plate (Sumitomo Bakelite Co., Ltd.) was further coated with laminin (20 µg/ml laminin, room temperature, 1 hour). Then, 100 µl of a medium (F12 medium containing 10% fetal bovine serum, 20 ng/ml NGF, 100 units/ml penicillin, and 100 µg/ml streptomycin) was added to each well. Thereafter, a dorsal root ganglion excised from a 7-day-old chicken embryo was inoculated into the resulting well, and the obtained mixture was then cultured under conditions consisting of 16 to 20 hours, 5% CO₂ and 37°C. Thereafter, the compounds described in Example 3 above were each added in different concentrations to the resulting culture, and the obtained mixture was then cultured for 1 hour. Thereafter, 2 units/ml mouse semaphorin 3A (Sema3A) was added to the culture, and the obtained mixture was further cultured for 1 hour. One hour after initiation of the culture, glutaraldehyde was added to the culture, such that it promptly could reach a final concentration of 1%. Then, the obtained mixture was then left at a room temperature for 15 minutes to fix a tissue section. Thereafter, the percentage of the retracted growth cone was measured under a microscope. A well, to which no Sema3A had been added, was used as a control. The percentage of growth cone retraction in a negative control group (to which neither the compound nor Sema3A was added) was defined as (A)%; the percentage of growth cone retraction in a positive control group (to which the compound had not been added but Sema3A was added) was defined as (B)%; and the percentage of growth cone retraction in a test group (to which both the compound and Sema3A were added) was defined as (C)%. The concentration of each compound that held the formula C = (A + B)/2 was defined as an IC₅₀ value. The results are shown below. From the obtained results, it is found that the compounds of Example 3 each strongly inhibit semaphorin 3A.

**[Table 1]**

| Compound | IC₅₀ (µg/ml) |
|---|---|
| SPF-3059-2 | <0.1 |
| SPF-3059-4 | 2.0 |
| SPF-3059-5 | 0.075 |
| SPF-3059-12 | 2.0 |
| SPF-3059-24 | 0.1 |
| SPF-3059-25 | 4.0 |
| SPF-3059-26 | 0.2 |

### Example 5

### Action of xanthone compound to suppress neurite elongation inhibitory activity of semaphorin 3A

A mass of Sema 3A-expressing COS7 cells and 7- or 8-day-old chicken embryo dorsal root ganglion were subjected to collagen gel co-culture (Neuroprotocols 4, 116, 1994), and whether or not the compounds of Example 3 each would exhibit an action to continuously inhibit Sema3 A was examined. The Sema3A-expressing COS7 cell mass was produced by the following method. That is, using FuGENE6 transfection reagent (Roche), 1 µg of a Sema3A-expressing plasmid was introduced into COS7 cells (100,000 cells/35 mm culture dish) that had been cultured overnight. 2.5 hours after initiation of the transfection, the COS7 cells were removed from the culture dish using trypsin, and they were then gathered by centrifugation. The gathered cells were suspended again in 200 µl of a medium. Thereafter, 20 µl of the cell suspension was added to the cap (inner side) of the culture dish, and the cap was then inverted, followed by performing a culture for 20 hours (hanging drop culture) (Cell, 78, 425, 1994). After completion of the culture, the coagulated COS7 cell (mass) was recovered on a medium, and it was then trimmed to a size of 0.5 mm diameter. This Sema 3A-expressing COS7 cell mass and the above described dorsal root ganglion were placed in 0.2% collagen gel with a distance of 0.5 to 1 mm therebetween, and this collagen gel was then cultured at 37°C in 5% CO₂ for 2 days in a medium containing each of the above-mentioned compounds in different concentrations. Subsequently, glutaraldehyde was added to the culture, such that it promptly could reach a final concentration of 1%. Then, the obtained mixture was then left at a room temperature for 1 hour to fix a tissue section. Thereafter, neurite elongation was measured under a microscope.

In the above described collagen gel, Sema3A was secreted from the COS7 cell mass into which the Sema3A-expressing plasmid had been introduced, and a concentration gradient was formed (in which the closer to the COS7 cell mass, the higher the concentration). When a medium containing no test compounds was used, the neurite could not be elongated in a direction in which the COS7 cell mass was present and thus the Sema3A concentration was high, and it was elongated only in an opposite direction. When the compounds of Example 3 were each added to a medium, neurite elongation was observed in a direction in which the Sema3 A-expressing COS7 cell mass was present.

A case in which neurites are elongated in a completely concentrical form, as in the case of the control group using Sema3A-non-expressing COS7 cells, is indicated with the symbol +++ (strong Sema3A inhibitory effect). A case in which neurites are elongated in a nearly concentrical form but their elongation to the side of the Sema3A-expressing COS7 cells is slightly suppressed is indicated with the symbol ++. A case in which neurite elongation in a semiluminar form to the side of the Sema3A-expressing COS7 cells is considerably suppressed is indicated with the symbol +. A case in which there is no neurite elongation to the side of the Sema 3A-expressing COS7 cells is indicated with - (no Sema3A inhibitory effect). The measurement results are shown below.

**[Table 2]**

| Compound | Test compound concentration (µg/ml) | | | | |
|---|---|---|---|---|---|
| | 0.5 | 1.0 | 2.0 | 6.0 | 20 |
| SPF-3059-2 | + | ++ | +++ | NT | NT |
| SPF-3059-4 | NT | NT | - | + | ++ |
| SPF-3059-5 | + | ++ | ++ | NT | NT |
| SPF-3059-12 | NT | NT | + | + | ++ |
| PBS (control) | - | - | - | - | - |

| | | | | | |
|---|---|---|---|---|---|
| (NT = not tested: not evaluated) | | | | | |

### Example 6

### Preparation Example 1

The following composition is suspended in 100 ml of sterile purified water, and the suspension is then adjusted to pH 7.0 in a concentration isotonized with tears, so as to prepare ophthalmic preparations.

| | |
|---|---|
| SPF-3059-5 | 50 mg |
| Potassium dihydrogen phosphate | suitable amount |
| Disodium hydrogen phosphate | suitable amount |
| Common salt | suitable amount |
| Benzethonium chloride | 10 mg |
| Sterile purified water | suitable amount |

### Example 7

### Preparation Example 2

The following composition is suspended in 100 ml of sterile purified water, and the suspension is then adjusted to pH 7.0 in a concentration isotonized with tears, so as to prepare ophthalmic preparations.

| | |
|---|---|
| SPF-3059-5 | 50 mg |
| Potassium dihydrogen phosphate | suitable amount |
| Disodium hydrogen phosphate | suitable amount |
| Common salt | suitable amount |
| Benzethonium chloride | 10 mg |
| Sterile purified water | suitable amount |

### Example 8

### Preparation Example 3

According to a common method for producing ophthalmic ointments, ophthalmic ointments can be prepared with the following formulation.

| | |
|---|---|
| SPF-3059-5 | 50 mg |
| Liquid paraffin | 10 g |
| White petrolatum | suitable amount |

### Example 9

### Preparation Example 4

According to a common method for producing ophthalmic ointments, ophthalmic ointments can be prepared with the following formulation.

| | |
|---|---|
| SPF-3059-5 | 50 mg |
| Liquid paraffin | 10 g |
| White petrolatum | suitable amount |

### Example 10

### Stability of xanthone compound in PBS (phosphate buffer)

SPF-3059-2, SPF-3059-5, SPF-3059-24 and SPF-3059-26, which are xanthone compounds included in the formula (1), and SPF-3059-1, SPF-3059-3, SPF-3059-9 and SPF-3059-30, which are xanthone compounds not included in the formula (1), were each dissolved in PBS (Dulbecco's PBS(-), phosphoric acid concentration: 10 mM) to a concentration of 100 µg/ml. (The structural formulae, production methods and physicochemical properties of the compounds SPF-3059-1, SPF-3059-3, SPF-3059-9 and SPF-3059-30 are described in International Publication WO02/09756 (the above-mentioned Patent Literature 1)). The obtained solutions were each preserved at 37°C. Four weeks later, the remaining amount of each compound was quantified by HPLC, and the remaining percentage was then obtained, so that the stability of the xanthone compound at 37°C in PBS was evaluated. HPLC conditions were the following. Column: Wakopak-Wakosil (registered trademark)-II5C18RS (4.6 mm in diameter x 150 mm, manufactured by Wako Pure Chemical Industries Ltd.), eluent A: 1% formic acid aqueous solution, eluent B: methanol, gradient: a linear gradient in which the percentage of solution B was 30% → 70% for 60 minutes, flow rate: 1.0 ml/min, and detection: absorbance at 260 nm.

The results obtained by evaluating the stability of the xanthone compounds in PBS are shown in Fig. 7. From the results shown in Fig. 7, it is found that all of the 4 types of xanthone compounds included in the formula (1) exhibited a remaining percentage of 90% or more. On the other hand, all of the 4 types of xanthone compounds that are not included in the formula (1) were decomposed in almost total amounts thereof.

### Example 11

### Stability of vinaxanthone in PBS (phosphate buffer)

Vinaxanthone (SPF-3059-5) and SPF-3059-1 were each dissolved in PBS (Dulbecco's PBS(-)) to a concentration of 100 µg/ml. The obtained solutions were preserved at 37°C, and the amount of the agent remaining in the solution was quantified by HPLC in individual time points from initiation of the preservation until 4 weeks after initiation of the preservation, so that a change over time in the remaining percentages was examined. The same HPLC conditions as those of Example 10 were applied.

The stability of vinaxanthone (SPF-3059-5) and SPF-3059-1 in PBS was evaluated. The results are shown in Fig. 8. From the results shown in Fig. 8, it is found that vinaxanthone (SPF-3059-5) that is a xanthone compound included in the formula (1) remained in PBS at 37°C at a remaining percentage of 96% or more until 4 weeks after initiation of the preservation, and thus that this compound was stable for 4 or more weeks. On the other hand, SPF-3059-1 was decomposed in an almost total amount thereof for approximately 1 week.

### Example 12

### Analysis regarding change in content of compound of the present invention in tears and in cornea

For the treatment of corneal nerve damage, eye drops administration of the agent is most preferable. Thus, it is important to analyze a change in the content of the agent in tears and/or in the cornea after eye drops administration of the agent. The analysis of a change in the content of the agent can be carried out, for example, according to the following test method.

### [Test method]

The compounds represented by the formula (1) of the present invention (for example, SPF-3059-5; hereinafter abbreviated to as the compound of the present invention) and the target compounds (compounds that are not included in the formula (1), such as the above-mentioned SPF-3059-1) were analyzed in terms of a change in the content thereof. That is, each compound was dissolved in PBS to a concentration of 0.1 mg/mL to 1 mg/mL. 10 µL each of a solution of the compound of the present invention, a solution of the target compound, or PBS alone was administered to one eye of each rat via eye drops administration. Thirty minutes, two hours, and 10 hours after the administration, 1 µL to 10 µL of tears was collected with a pipette, and the rats were then subjected to euthanasia. The eyeball was excised from each rat, and the cornea was then collected.

The content of each compound in tears and/or in the cornea was measured by LC-MS. 0.1 M HCl was added to the sample, and thereafter, the obtained mixture was stirred in the case of tears, or was homogenized in the case of the cornea. Thereafter, ethyl acetate was added to the resultant, and the obtained mixture was then fully stirred for centrifugation. Then, an upper layer was collected. Ethyl acetate was further added to a lower layer, and the above described operations were repeatedly carried out on the obtained mixture. Upper layers from three times of operations were gathered, and a sample obtained by drying the gathered layer with nitrogen gas was then used in LC-MS measurement.

### Industrial Applicability

The xanthone compound having a semaphorin 3A inhibitory activity of the present invention is effective as a therapeutic or preventive agent for sensory neuropathy caused by corneal disease or corneal surgery. Specifically, the present xanthone compound can be effectively used as a therapeutic or preventive agent for sensory neuropathy caused by the corneal sensory nerve damaged due to a corneal disease such as keratitis, leukoma, corneal infection, corneal degeneration, corneal dystrophy, corneal stromal dystrophy bullous keratopathy, keratoconus, corneal decompensation, corneal ulcer, neuroparalytic keratopathy, diabetic keratopathy, chemical burns of the cornea, or thermal burns of the cornea; or for sensory neuropathy caused by the corneal sensory nerve damaged due to corneal surgery such as keratoplasty, myopia correction surgery, and corneal surgery that targets the cornea required for the treatment of eye disease or corneal injury. In addition, the xanthone compound having a semaphorin 3 A inhibitory activity of the present invention can also be effectively used as a promoter for the regeneration of the corneal sensory nerve.

Moreover, the xanthone compound used in the present invention is chemically extremely stable in an aqueous solution such as a phosphate buffer. Thus, when the present xanthone compound is administered in the form of an eye drop, it is stable in tears or cornea. Accordingly, the present xanthone compound is extremely preferable as a therapeutic or preventive agent for sensory neuropathy caused by corneal disease or corneal surgery, and also as a promoter for the regeneration of the corneal sensory nerve.

## Claims

1. An agent comprising, as an active ingredient, a compound represented by the following formula (1) or a pharmaceutically acceptable salt thereof: 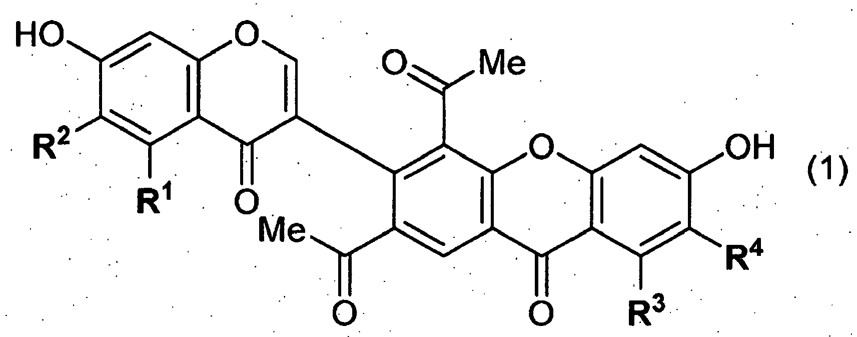 wherein R¹ represents a hydrogen atom or a carboxyl group; R² represents a hydrogen atom or a hydroxyl group; R³ represents a hydrogen atom or a carboxyl group; and R⁴ represents a hydrogen atom or a hydroxyl group;
for use in a method that is:
(i) a method for treatment or prevention of sensory neuropathy caused by cornea disease or corneal surgery; or
(ii) a method for promoting regeneration of corneal sensory nerve.
wherein the compound represented by the formula (1) or a pharmaceutically acceptable salt thereof is parenterally administered topically into eyes.

2. The agent for use according to claim 1, wherein the corneal sensory nerve has been disconnected or damaged in the sensory neuropathy.

3. The agent for use according to claim 1 or 2, wherein the method is a method for promoting regeneration of corneal sensory nerve and a method for treatment or prevention of sensory neuropathy caused by corneal disease or corneal surgery.

4. The agent for use according to any one of claims 1 to 3, wherein, in the formula (1), at least one of R² and R⁴ represents a hydroxyl group.

5. The agent for use according to claim 4, wherein, in the formula (1), R² and R⁴ each represent a hydroxyl group.

6. The agent for use according to any one of claims 1 to 5, wherein, in the formula (1), at least one of R¹ and R³ represents a carboxyl group.

7. The agent for use according to claim 6, wherein, in the formula (1), R³ represents a carboxyl group.

8. The agent for use according to any one of claims 1 to 3, wherein, in the formula (1), R¹ and R³ each represent a carboxyl group, and R² and R⁴ each represent a hydroxyl group.

9. The agent for use according to any one of claims 1 to 8, wherein the sensory neuropathy is caused by corneal disease and the corneal disease is keratitis, leukoma, corneal infection, corneal degeneration, corneal dystrophy, corneal stromal dystrophy, bullous keratopathy, keratoconus, corneal decompensation, corneal ulcer, neuroparalytic keratopathy, diabetic keratopathy, chemical burns of the cornea, or thermal burns of the cornea.

10. The agent for use according to any one of claims 1 to 8, wherein the sensory neuropathy is caused by corneal surgery and the corneal surgery is keratoplasty.

11. The agent for use according to any one of claims 1 to 8, wherein the sensory neuropathy is caused by corneal surgery and the corneal surgery is myopia correction surgery.

12. The agent for use according to any one of claims 1 to 8, wherein the sensory neuropathy is caused by corneal surgery and the corneal surgery targets the cornea required for the treatment of eye disease or corneal injury.

13. The agent for use according to any one of claims 1 to 12, wherein the sensory neuropathy caused by the corneal sensory nerve damaged due to corneal disease or corneal surgery is imperceptions.

14. The agent for use according to any one of claims 1 to 12, wherein the sensory neuropathy caused by the corneal sensory nerve damaged due to corneal disease or corneal surgery is dry eyes.

15. The agent for use according to any one of claims 1 to 14, which is in the form of an ophthalmic preparation.

## Patentansprüche

1. Ein Mittel, umfassend als einen Wirkstoff, eine Verbindung, dargestellt durch die folgende Formel (1) oder ein pharmazeutisch verträgliches Salz davon: 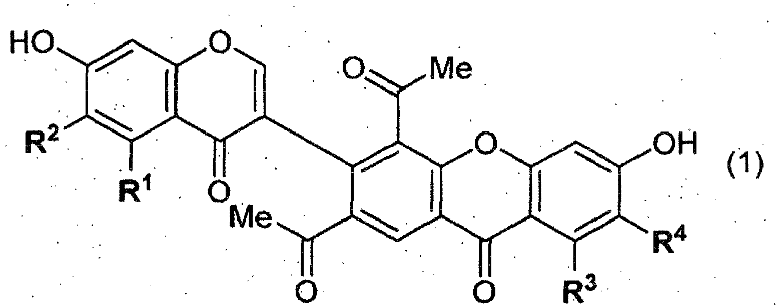 wobei R¹ ein Wasserstoffatom oder eine Carboxylgruppe darstellt; R² ein Wasserstoffatom oder eine Hydroxylgruppe darstellt; R³ ein Wasserstoffatom oder eine Carboxylgruppe darstellt; und R⁴ ein Wasserstoffatom oder eine Hydroxylgruppe darstellt;
zur Verwendung in einem Verfahren, wobei es sich um:
(i) ein Verfahren zur Behandlung oder Vorbeugung von sensorischer Neuropathie, verursacht durch eine Hornhauterkrankung oder einen Hornhauteingriff; oder
(ii) ein Verfahren zur Förderung der Regeneration eines sensorischen Hornhautnervs handelt, wobei die Verbindung, dargestellt durch die Formel (1) oder ein pharmazeutisch verträgliches Salz davon parenteral, topisch in die Augen verabreicht wird.

2. Das Mittel zur Verwendung nach Anspruch 1, wobei bei der sensorischen Neuropathie der sensorische Hornhautnerv durchtrennt oder beschädigt wurde.

3. Das Mittel zur Verwendung nach Anspruch 1 oder 2, wobei es sich bei dem Verfahren um ein Verfahren zur Förderung der Regeneration eines sensorischen Hornhautnervs und ein Verfahren zur Behandlung oder Vorbeugung von sensorischer Neuropathie, verursacht durch eine Hornhauterkrankung oder einen Hornhauteingriff, handelt.

4. Das Mittel zur Verwendung nach einem der Ansprüche 1 bis 3, wobei in der Formel (1) mindestens einer aus R² und R⁴ eine Hydroxylgruppe darstellt.

5. Das Mittel zur Verwendung nach Anspruch 4, wobei in der Formel (1) R² und R⁴ jeweils eine Hydroxylgruppe darstellen.

6. Das Mittel zur Verwendung nach einem der Ansprüche 1 bis 5, wobei in Formel (1) mindestens einer aus R¹ und R³ eine Carboxylgruppe darstellt.

7. Das Mittel zur Verwendung nach Anspruch 6, wobei in der Formel (1) R³ eine Carboxylgruppe darstellt.

8. Das Mittel zur Verwendung nach einem der Ansprüche 1 bis 3, wobei in der Formel (1) R¹ und R³ jeweils eine Carboxylgruppe darstellen und R² und R⁴ jeweils eine Hydroxylgruppe darstellen.

9. Das Mittel zur Verwendung nach einem der Ansprüche 1 bis 8, wobei die sensorische Neuropathie durch eine Hornhauterkrankung verursacht wird und es sich bei der Hornhauterkrankung um Keratitis, Leukom, Hornhautinfektion, Hornhautdegeneration, Hornhautdystrophie, stromale Hornhautdystrophie, bullöse Keratopathie, Keratokonus, Hornhautdekompensation, Hornhautgeschwür, neuroparalytische Keratopathie, diabetische Keratopathie, chemische Verbrennungen der Hornhaut oder thermische Verbrennungen der Hornhaut handelt.

10. Das Mittel zur Verwendung nach einem der Ansprüche 1 bis 8, wobei die sensorische Neuropathie durch einen Hornhauteingriff verursacht wird und es sich bei dem Hornhauteingriff um Keratoplastik handelt.

11. Das Mittel zur Verwendung nach einem der Ansprüche 1 bis 8, wobei die sensorische Neuropathie durch einen Hornhauteingriff verursacht wird und es sich bei dem Hornhauteingriff um einen Eingriff zur Korrektur der Myopie handelt.

12. Das Mittel zur Verwendung nach einem der Ansprüche 1 bis 8, wobei die sensorische Neuropathie durch einen Hornhauteingriff verursacht wird und der Hornhauteingriff auf die Hornhaut, die für die Behandlung einer Augenerkrankung oder Hornhautverletzung benötigt wird, gerichtet ist.

13. Das Mittel zur Verwendung nach einem der Ansprüche 1 bis 12, wobei es sich bei der sensorischen Neuropathie, die durch den sensorischen Hornhautnerv, der aufgrund einer Hornhauterkrankung oder eines Hornhauteingriffs geschädigt wurde, verursacht wird, um Wahrnehmungslosigkeit handelt.

14. Das Mittel zur Verwendung nach einem der Ansprüche 1 bis 12, wobei es sich bei der sensorischen Neuropathie, die durch den sensorischen Hornhautnerv, der aufgrund einer Hornhauterkrankung oder eines Hornhauteingriffs geschädigt wurde, verursacht wird, um trockene Augen handelt.

15. Das Mittel zur Verwendung nach einem der Ansprüche 1 bis 14, das in Form eines Augenpräparats vorliegt.

## Revendications

1. Agent comprenant, en tant que principe actif, un composé représenté par la formule suivante (1) ou un sel pharmaceutiquement acceptable de celui-ci : 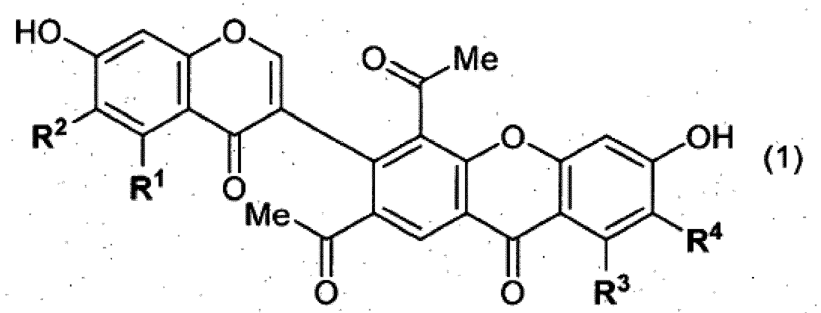 dans lequel R¹ représente un atome d'hydrogène ou un groupe carboxyle ; R² représente un atome d'hydrogène ou un groupe hydroxyle ; R³ représente un atome d'hydrogène ou un groupe carboxyle ; et R⁴ représente un atome d'hydrogène ou un groupe hydroxyle ;
destiné à être utilisé dans un procédé qui est :
(i) un procédé pour traiter ou prévenir une neuropathie sensorielle causée par une maladie de la cornée ou une chirurgie cornéenne ; ou
(ii) un procédé pour favoriser la régénération du nerf sensoriel cornéen ;
dans lequel le composé représenté par la formule (1) ou un sel pharmaceutiquement acceptable de celui-ci, est administré par voie parentérale et topique dans les yeux.

2. Agent destiné à être utilisé selon la revendication 1, dans lequel le nerf sensoriel cornéen a été déconnecté ou lésé dans la neuropathie sensorielle.

3. Agent destiné à être utilisé selon la revendication 1 ou 2, dans lequel le procédé est un procédé pour favoriser la régénération du nerf sensoriel cornéen et un procédé pour traiter ou prévenir une neuropathie sensorielle causée par une maladie de la cornée ou une chirurgie cornéenne.

4. Agent destiné à être utilisé selon l'une quelconque des revendications 1 à 3, dans lequel, dans la formule (1), au moins un de R² et R⁴ représente un groupe hydroxyle.

5. Agent destiné à être utilisé selon la revendication 4, dans lequel, dans la formule (1), R² et R⁴ représentent chacun un groupe hydroxyle.

6. Agent destiné à être utilisé selon l'une quelconque des revendications 1 à 5, dans lequel, dans la formule (1), au moins un de R¹ et R³ représente un groupe carboxyle.

7. Agent destiné à être utilisé selon la revendication 6, dans lequel, dans la formule (1), R³ représente un groupe carboxyle.

8. Agent destiné à être utilisé selon l'une quelconque des revendications 1 à 3, dans lequel, dans la formule (1), R¹ et R³ représentent chacun un groupe carboxyle, et R² et R⁴ représentent chacun un groupe hydroxyle.

9. Agent destiné à être utilisé selon l'une quelconque des revendications 1 à 8, dans lequel la neuropathie sensorielle est causée par une maladie de la cornée, et la maladie de la cornée est une kératite, un leucome, une infection cornéenne, une dégénération cornéenne, une dystrophie cornéenne, une dystrophie cornéenne stromale, une kératopathie bulleuse, un kératocône, une décompensation cornéenne, un ulcère cornéen, une kératopathie neuroparalytique, une kératopathie diabétique, des brûlures chimiques ou thermiques de la cornée.

10. Agent destiné à être utilisé selon l'une quelconque des revendications 1 à 8, dans lequel la neuropathie sensorielle est causée par une chirurgie cornéenne, et la chirurgie cornéenne est une kératoplastie.

11. Agent destiné à être utilisé selon l'une quelconque des revendications 1 à 8, dans lequel la neuropathie sensorielle est causée par une chirurgie cornéenne, et la chirurgie cornéenne est une chirurgie de correction de la myopie.

12. Agent destiné à être utilisé selon l'une quelconque des revendications 1 à 8, dans lequel la neuropathie sensorielle est causée par une chirurgie cornéenne, et la chirurgie cornéenne cible la cornée pour le traitement d'une maladie des yeux, ou d'une blessure de la cornée.

13. Agent destiné à être utilisé selon l'une quelconque des revendications 1 à 12, dans lequel la neuropathie sensorielle causée par le nerf sensoriel cornéen lésé par suite d'une maladie de la cornée ou d'une chirurgie cornéenne se manifeste par un défaut de perception.

14. Agent destiné à être utilisé selon l'une quelconque des revendications 1 à 12, dans lequel la neuropathie sensorielle causée par le nerf sensoriel cornéen lésé par suite d'une maladie de la cornée ou d'une chirurgie cornéenne se manifeste par des yeux secs.

15. Agent destiné à être utilisé selon l'une quelconque des revendications 1 à 14, qui se présente sous la forme d'une préparation ophtalmique.
